(19) Europäisches Patentamt
European Patent Office
Office européen des brevets

(11) **EP 2 996 686 B1**

(12) **FASCICULE DE BREVET EUROPEEN**

(45) Date de publication et mention
de la délivrance du brevet:
**03.04.2019 Bulletin 2019/14**

(21) Numéro de dépôt: **14727731.3**

(22) Date de dépôt: **13.05.2014**

(51) Int Cl.:
*A61K 31/19* (2006.01)     *A61K 31/194* (2006.01)
*A61K 31/198* (2006.01)    *A61K 31/353* (2006.01)
*A61K 31/375* (2006.01)    *A61K 9/00* (2006.01)
*A61K 9/08* (2006.01)      *A61K 47/18* (2017.01)
*A61P 19/02* (2006.01)     *A61P 29/00* (2006.01)
*A61P 35/02* (2006.01)     *A61P 35/00* (2006.01)

(86) Numéro de dépôt international:
**PCT/EP2014/059781**

(87) Numéro de publication internationale:
**WO 2014/184198 (20.11.2014 Gazette 2014/47)**

(54) **COMPOSITION ORALE A BASE DE (+)-CATECHINE**

ORALE ZUSAMMENSETZUNG MIT (+)-CATECHIN

ORAL COMPOSITION COMPRISING (+)-CATECHIN

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **17.05.2013 BE 201300350**

(43) Date de publication de la demande:
**23.03.2016 Bulletin 2016/12**

(73) Titulaire: **Valore**
**7180 Seneffe (BE)**

(72) Inventeurs:
• **NIEBES, Paul**
**B-1390 Grez-Doiceau (BE)**
• **MAY, Bronislav, Henric**
**B-3090 Overijse (BE)**
• **RACHIDI, Saïd**
**B-7022 Hyon (BE)**
• **ESTAGER, Julien**
**B-2550 Kontich (BE)**
• **SCHOENTJES, Bruno**
**F-92310 Sèvres (FR)**

(74) Mandataire: **Paemen, Liesbet R.J. et al**
**De Clercq & Partners cvba**
**Edgard Gevaertdreef 10 a**
**9830 Sint-Martens-Latem (BE)**

(56) Documents cités:
CH-A5- 665 634          CN-A- 1 943 572
GB-A- 2 306 321          US-A- 4 285 964
US-A1- 2003 130 201

• WEYANT ET AL.: "(+)-CATECHIN INHIBITS
INTESTINAL TUMOR FORMATION AND
SUPPRESSES FOCAL ADHESION KINASE
ACTIVATION IN THE MIN/+ MOUSE", CANCER
RESEARCH, vol. 61, 2001, pages 118-125,
XP002719184, cité dans la demande

**Description**

La présente invention se rapporte à une composition gastro-entérique à administration orale comprenant un composé de (+)-catéchine monomérique et au moins un acide aminé basique, destinée aux mammifères, et en particulier à l'être humain.

Les polyphénols constituent une famille de molécules organiques largement présente dans le règne végétal. Ils sont caractérisés, comme l'indique le nom, par la présence de plusieurs groupements phénoliques associés en structures généralement de haut poids moléculaire. Ces composés sont les produits du métabolisme secondaire des plantes.

En particulier, la (+)-catéchine sous sa forme monomérique est directement obtenue à partir de l'extrait de *l'Uncaria Gambir.*

Une telle composition est déjà connue par exemple du document US 4 285 964 dans le cadre du traitement de maladies chroniques de type dégénératives des tissus conjonctifs. Parmi ces maladies dégénératives, dont les plus connues et les plus répandues chez l'être humain sont par exemple l'arthrose, la chondromalacie et la parodontose.

Selon le document US 4 285 964, la composition comprenant un composé de (+)-catéchine monomérique et au moins un acide aminé basique est un médicament dans lequel la substance active est la (+)-catéchine monomérique

Ce document divulgue par ailleurs que, pour pouvoir traiter ces maladies, il est important de pouvoir administrer ce composé sur le site où la substance active doit agir, c'est-à-dire dans les articulations atteintes par la maladie.

Le brevet US 4 285 964 a donc pour but principal de former une composition injectable qui puisse être directement injectée dans le corps, par exemple dans les articulations.

Cet objectif est atteint par la formation d'un double sel de (+)-catéchine monomérique qui, en n'outre, n'est pas caractérisé exactement puisqu'il s'agit d'un mélange équimoléculaire de (+)-catéchine, de lysine et d'acide chlorhydrique, afin d'obtenir une solubilisation de la (+)-catéchine en milieu aqueux, à pH physiologique dans le but de disposer d'une forme injectable, l'ajout de l'acide permettant de moduler la solubilité de ce double sel et d'ajuster le pH de ce dernier à la valeur physiologique (pH = 7,2 - 7,4).

En effet, ce document met en évidence que la solubilité dans une solution aqueuse (comme de l'eau au pH physiologique) de la (+)-catéchine est élevée, jusqu'à 400g/L, lorsque cette dernière forme un double sel en présence d'un acide aminé basique comme la L-arginine ou la L-lysine et d'un acide, par exemple comme l'HCl.

Cette solubilité améliorée en milieu aqueux est plus intéressante pour une administration de type parentérale que l'utilisation de la (+)-catéchine monomérique pure qui présente une solubilité dans l'eau est limitée à 1g/L.

Le document US 4 285 964 extrapole au passage la composition injectable susdite pour obtenir une formulation orale par séchage de la composition injectable.

Toutefois, ce document ne donne pas d'indication quant à la stabilité et aux capacités d'assimilation par l'organisme *in vivo* de la composition orale décrite ci-dessus.

Le document CH 665 634 divulgue des composés complexes de (+)-catéchine et du sel de double addition de L-lysine et d'un acide minéral et/ou organique afin d'obtenir un composé plus stable et plus soluble, et des procédés pour leur préparation. Ce document ne donne pas d'indication quant à la biodisponibilité de la (+)catéchine par voie orale. Les composés du document CH 665 634 présentent un ratio d'équivalence molaire de 1 :1 entre la (+)catéchine monomérique par rapport à au moins un acide aminé basique.

Dès lors, au vu de l'état de la technique actuel, il existe un besoin de disposer d'une composition gastro-entérique à administration orale à base de (+)-catéchine monomérique qui soit assimilable par voie orale et qui reste stable une fois ingérée.

Dans ce but, la présente invention prévoit une composition caractérisée en ce que ledit composé est sous forme de complexe et présente un ratio d'équivalence molaire de ladite (+)-catéchine monomérique par rapport audit au moins un acide aminé basique compris entre 1 : 1,5 et 1 : 2,5 (dénommé ci-après comme un complexe [C :AA/1 : 1,5 - 1 : 2,5], C pour (+)-catéchine monomérique et AA pour acide aminé basique).

En effet, dans le cadre de la présente invention, il a été noté de façon tout à fait surprenante que le complexe de (+)-catéchine monomérique avec au moins un acide aminé basique dans un ratio d'équivalence molaire spécifique compris entre 1,5 : 1 et 1 : 2,5 est stable une fois ingéré et présente une biodisponibilité par voie orale élevée. Cette biodisponibilité élevée par voie orale est rendue possible par la stabilité *in vivo* du complexe selon l'invention.

On pense en effet que la présence dudit au moins un acide aminé basique lié à la (+)-catéchine monomérique permet au complexe de traverser facilement la paroi intestinale, il en résulte donc une proportion en (+)-catéchine monomérique libre dans le sang plus élevée.

A partir des résultats présentés dans le cadre de la présente invention, on observe que la biodisponibilité élevée par voie orale d'un gramme de (+)-catéchine monomérique sous forme d'un complexe atteint une valeur d'au moins 900 ng h/ml (54000 ng min/ml) chez l'homme. Au vu des résultats de la présente invention, on entend donc par biodisponibilité élevée chez l'homme, une biodisponibilité au moins égale à 900 ng h/ml (54000 ng min/ml) pour une administration orale d'un gramme.

Les résultats obtenus par les inventeurs de la présente demande de brevet montrent que la biodisponibilité mesurée chez l'homme pour un gramme de (+)-catéchine ingérée sous la forme de complexe de (+)-catéchine monomérique et d'au moins un acide aminé basique dans un ratio d'équivalence molaire compris entre 1 : 1,5 et 1 : 2,5, est d'au moins 900 ng h/ml (54000 ng min/ml).

A titre illustratif, il est observé dans le cadre de la présente invention que la biodisponibilité par voie orale chez l'être humain de la (+)-catéchine ingérée sous la forme de complexe de (+)-catéchine monomérique est bien supérieure [1263 ± 88 ng h/ml (75780 ng min/ml) pour 0,920 g de (+)-catéchine monomérique ingérée sous forme de 1,5g de complexe] à celle de la (+)-catéchine monomérique ingérée seule [832 ± 150 ng h/ml (49920 ng min/ml) pour une quantité équivalente de 0,920 g d'une prise orale de (+)-catéchine monomérique pure], soit +52% d'augmentation de biodisponibilité grâce au complexe.

Cette caractéristique est d'autant plus inattendue qu'il a été démontré dans le cadre de la présente invention que la (+)-catéchine monomérique pure présente une solubilité dans l'eau 400 fois moins élevée que celle mesurée lorsqu'elle est solubilisée sous une forme de complexe formé avec au moins un acide aminé basique

En effet la solubilité des sels de lysine de la (+)-catéchine atteint à 20°C 400g par litre d'eau alors que la (+)-catéchine elle-même présente dans les mêmes conditions une solubilité de 0,9 g par litre.

Dès lors que la composition selon l'invention présente une biodisponibilité élevée par voie orale, cette dernière permet alors une assimilation à des doses réduites, ce qui constitue d'une part une perspective de gain en termes de coût de traitement, dès lors qu'une dose moindre doit être absorbée, et, d'autre part, une diminution des effets secondaires liés à ce traitement, en particulier dans le cadre d'un traitement de longue durée, dès lors que l'on sait que la (+)-catéchine monomérique, même si elle est généralement bien tolérée dans l'organisme, .

En outre, il a été observé que :

- l'utilisation de (+)-catéchine administrée oralement sous forme d'un complexe [C: AA/1 : 1,5 - 1 : 2,5] donne une concentration plasmatique maximale en (+)-catéchine monomérique libre c (max) plus que doublée par rapport à celle obtenue avec la même dose de (+)-catéchine monomérique pure ingérée dans les mêmes conditions : c (max) de 571 ng/ml por 1g de (+)-catéchine ingérée sous forme d'un complexe contre 280 ng/ml pour 1g de (+)-catéchine monomérique ingérée telle quelle, soit une augmentation de + 103 % ; et que
- les concentrations plasmatiques maximales plus que doublées sont atteintes [T (max)] plus de deux fois plus rapidement lorsque la (+)-catéchine est ingérée sous forme d'un complexe [C : AA/1 : 1,5 - 1 : 2,5] plutôt que sous forme de (+)-catéchine monomérique seule : T (max) de 30 min contre 80 min, soit une augmentation de + 166%.

Ainsi, outre la biodisponibilité élevée *per* os du complexe selon l'invention, il est aussi démontré dans la présente demande que, dans le cas d'une absorption par voie orale, une proportion en (+)-catéchine monomérique libre dans le sang est maintenue de manière différée dans le temps, ce qui permet d'envisager une action curative et/ou préventive améliorée de la (+)-catéchine monomérique lorsqu'elle est administrée *per os.*

De préférence, ledit ratio d'équivalence molaire est inférieur ou égal à 1 : 2,5.

En particulier ledit ratio d'équivalence molaire est inférieur à 1 : 2,5, plus particulièrement inférieur ou égal à 1 : 2.

De manière préférentielle, ledit ratio d'équivalence molaire est inférieur à 1 : 2.

Alternativement, le ratio d'équivalence molaire est compris entre 1 : 1,5 et 1 : 2

Avantageusement, ledit ratio d'équivalence molaire est supérieur ou égal à 1,00 : 1,50 et inférieur ou égal à 1,00 : 2,50.

De préférence, ledit ratio d'équivalence molaire est supérieur ou égal à 1,00 : 1,50 et inférieur ou égal à 1,00 : 2,00.

De préférence, ledit ratio d'équivalence molaire est supérieur ou égal à 1,00 : 1,50 et inférieur ou égal à 1,00 : 2,50.

De préférence, ledit au moins un acide aminé basique est de la lysine.

Dans une forme particulière de réalisation de la présente invention, ledit complexe est un complexe comprenant deux molécules de lysine pour une molécule de (+)-catéchine monomérique.

Le complexe de (+)-catéchine avec deux lysines est de loin dans toutes les expériences la meilleure combinaison pour améliorer la biodisponibilité de la (+)-catéchine monomérique par voie orale

Dans une autre forme particulière de réalisation de la présente invention, ledit complexe est un complexe comprenant deux molécules d'arginine pour une molécule de (+)-catéchine monomérique.

Dans une autre forme préférentielle, ledit complexe est un complexe comprenant une molécule de lysine et une molécule d'arginine pour une molécule de (+)-catéchine monomérique.

De préférence, la composition selon l'invention est caractérisée en ce que ledit complexe est sous forme de sel dudit complexe, ledit sel comprenant ledit complexe, ledit complexe comprenant au moins un proton issu d'au moins un acide et au moins un anion issu dudit au moins un acide, ledit sel présentant ledit proton en quantité équimolaire par rapport à celle en acide aminé basique.

De cette façon, le sel de complexe est défini par les formules suivantes :

- [C: AA: H$^+$: A$^-$/1: x: x: x], dans le cas d'un acide monofonctionnel comme le HCl; ou
- [C: xAA: xH$^+$: x/yA$^y$-], dans le cas d'un acide,

H$^+$ représentant le proton de l'acide,
A$^-$ représentant l'anion de l'acide,
x représentant le nombre d'équivalent molaire en AA,
y représentant la charge portée par l'anion.

Avantageusement, ledit sel est le chlorhydrolysinate de (+)-catéchine monomérique.

Avantageusement, ledit au moins un acide est de préférence choisi parmi l'acide ascorbique, l'acide acétique, l'acide citrique et l'acide chlorhydrique. Très avantageusement, ledit acide est de l'acide ascorbique.

Le rôle de l'acide ascorbique est celui d'un complément vitaminique. De plus, cet acide étant aussi un antioxydant, il joue donc un rôle antioxydant synergique par rapport à celui de la (+)-catéchine.

Cette addition d'un acide permet en outre d'améliorer la biodisponibilité de la (+)-catéchine par voie orale. Cette observation est expliquée par l'état plus stable de la (+)-catéchine en milieu acide.

Dans un mode particulier de réalisation, ladite composition est caractérisée en ce qu'elle comprend en outre un ou plusieurs excipients biocompatibles.

De préférence, la teneur en complexe de (+)-catéchine avec ledit acide aminé basique est comprise entre 15 et 95% en poids par rapport au poids total de ladite composition, de préférence entre 60 et 90%, avantageusement de 65 à 85%.

De préférence, la composition est sous forme liquide ou sous forme solide, de préférence sous forme solide hydrosoluble, en particulier sous forme de poudre, de comprimé, ou de tablette.

Avantageusement, la composition sous forme liquide présente, dans une solution 0,01 molaireà 25°C, un pH égal ou supérieur à 3 (pH théorique d'un sel C:L dans un rapport 1 :0,5), de préférence compris entre 4 et 11, de manière avantageuse entre 4,5 et 9.

Eventuellement, la composition selon l'invention est une composition solide, de pH égal ou supérieur à 3, de préférence compris entre 4 et 11, de manière avantageuse entre 4,5 et 9, lorsqu'elle est mise en solution à 0,01 M à 25°C, comprenant de la (+)-catéchine monomérique et ledit au moins un acide aminé basique, et éventuellement au moins un acide, en tant que précurseurs dudit complexe ou dudit sel du complexe, comme préparation combinée pour une utilisation par voie orale simultanée, ledit complexe se formant post-administration orale.

La composition solide selon l'invention comprend la (+)-catéchine monomérique et ledit au moins un acide aminé basique dans un ratio d'équivalence molaire compris entre 1 : 1,5 et 1 : 2,5.

Ledit ratio d'équivalence molaire est inférieur ou égal à 1 : 2,5.

En particulier ledit ratio d'équivalence molaire est inférieur à 1 : 2,5, plus particulièrement inférieur ou égal à 1 : 2.

De manière préférentielle, ledit ratio d'équivalence molaire est inférieur à 1 : 2.

Alternativement, le ratio d'équivalence molaire est compris entre 1 : 1,5 et 1 : 2.

Avantageusement, ledit ratio d'équivalence molaire est supérieur ou égal à 1,00 : 1,50 et inférieur ou égal à 1,00 : 2,50.

De préférence, ledit ratio d'équivalence molaire est supérieur ou égal à 1,00 : 1,50 et inférieur ou égal à 1,00 : 2,00.

De préférence, ledit ratio d'équivalence molaire est supérieur ou égal à 1,00 : 1,50 et inférieur ou égal à 1,00 : 2,50.

La (+)-catéchine monomérique et ledit au moins un acide aminé basique sont des précurseurs qui participent, en milieu aqueux, à une réaction de complexation pour former le complexe selon l'invention.

Dans ce cas, le complexe est formé *in vivo* dans le tractus gastro-intestinal au contact de l'eau de la salive ou de l'eau contenue dans le bol stomacal.

De manière alternative, la composition selon l'invention est une composition solide comprenant de la (+)-catéchine monomérique et ledit au moins un acide aminé basique, et éventuellement au moins un acide, en tant que précurseurs dudit complexe ou dudit sel du complexe, comme préparation combinée pour une utilisation par voie orale simultanée en solution dans une phase aqueuse, ledit complexe se formant pré-administration orale, ladite (+)-catéchine monomérique et ledit au moins un acide aminé basique étant présents dans un ratio d'équivalence molaire compris entre 1 : 1,5 et 1 : 2,5.

Ledit ratio d'équivalence molaire est inférieur ou égal à 1 : 2,5.

En particulier ledit ratio d'équivalence molaire est inférieur à 1 : 2,5, plus particulièrement inférieur ou égal à 1 : 2.

De manière préférentielle, ledit ratio d'équivalence molaire est inférieur à 1 : 2.

Alternativement, le ratio d'équivalence molaire est compris entre 1 : 1,5 et 1 : 2.

Avantageusement, ledit ratio d'équivalence molaire est supérieur ou égal à 1,00 : 1,50 et inférieur ou égal à 1,00 : 2,50.

De préférence, ledit ratio d'équivalence molaire est supérieur ou égal à 1,00 : 1,50 et inférieur ou égal à 1,00 : 2,00.

De préférence, ledit ratio d'équivalence molaire est supérieur ou égal à 1,00 : 1,50 et inférieur ou égal à 1,00 : 2,50.

De cette façon, le complexe est formé dès lors que la composition est mise en solution aqueuse, c'est-à-dire avant administration orale.

En particulier, la présente invention porte sur une composition alimentaire ou thérapeutique, curative ou prophylactique.

D'autres formes de réalisation de la composition suivant l'invention sont indiquées dans les revendications annexées.

La présente invention porte en outre sur la composition selon l'invention, pour une utilisation pour le traitement par voie orale, préventif et/ou curatif, du cancer de préférence du cancer hépatocellulaire.

Avantageusement, la présente invention porte en outre sur la composition selon l'invention, pour une utilisation pour le traitement par voie orale, préventif et/ou curatif des leucémies.

Très avantageusement, la présente invention porte en outre sur la composition selon l'invention, pour une utilisation par voie orale pour le traitement préventif et/ou curatif des myélomes.

Eventuellement, la présente invention porte en outre sur la composition selon l'invention, pour une utilisation par voie orale pour le traitement préventif et/ou curatif des lymphomes.

Eventuellement, la présente invention porte en outre sur la composition selon l'invention, pour une utilisation dans le cadre du traitement préventif et/ou curatif des cancers du foie, de la prostate, du sein, de l'utérus, des testicules, de la vessie, des reins, des poumons, des bronches, des os, de la bouche, de l'oesophage, de l'estomac, du pancréas, colon-rectal.

D'autres formes de réalisation de la composition suivant l'invention pour le traitement par voie orale, préventif et/ou curatif, du cancer sont indiquées dans les revendications annexées.

La présente invention porte en outre sur la composition selon l'invention, pour une utilisation par voie orale dans le cadre du traitement préventif et/ou curatif des maladies inflammatoires et/ou dégénératives, en particulier de l'arthrose.

Cette utilisation est rendue possible grâce à l'action anti-inflammatoire reconnue de la (+)-catéchine monomérique.

D'autres formes de réalisation de la composition suivant l'invention pour le traitement par voie orale, préventif et curatif, des maladies inflammatoires sont indiquées dans les revendications annexées.

La présente invention porte en outre sur la composition selon l'invention, pour une utilisation par voie orale dans le cadre du traitement préventif et/ou curatif du vieillissement cellulaire.

Cette utilisation est rendue possible grâce à l'action antioxydante reconnue de la (+)-catéchine monomérique.

D'autres formes de réalisation de la composition selon l'invention pour le traitement par voie orale, préventif et/ou curatif, du vieillissement cellulaire sont indiquées dans les revendications annexées.

La présente invention porte en outre sur la composition selon l'invention, pour une utilisation par voie orale dans le cadre du traitement préventif et/ou curatif de carences en collagène.

Cette utilisation est rendue possible grâce à l'action stimulatrice de production de collagène et de la protection de ces fibres par la (+)-catéchine monomérique et ses sels telle que démontrée dans la présente demande.

En particulier, la présente composition selon l'invention vise le traitement préventif et/ou curatif des maladies génétiques du tissu conjonctif telles que l'ostéogénèse imparfaite, les maladies de Marfan, d'Ehlers-Danlos, les maladies de Cutis Laxa, la sclérodermie systémique, et autres maladies du tissu conjonctif où le collagène et/ou l'élastine qui apparaissent être anormalement solubles tel que c'est le cas également dans la parodontose-parodontite, la sclérodermie, les vergetures, les problèmes de réparation tissulaire ou de lésions du tissu conjonctif provoquées par l'irradiation, la radiothérapie ou l'exposition prolongée aux rayons du soleil.

D'autres formes de réalisation de la composition suivant l'invention pour le traitement par voie orale, préventif et/ou curatif, de carences en collagène sont indiquées dans les revendications annexées.

La présente invention porte également sur la composition selon l'invention, pour une utilisation par voie orale dans le cadre du traitement préventif et/ou curatif de l'ulcère peptique et des allergies gastro-intestinales.

Cette utilisation est rendue possible grâce à l'action protectrice de la composition selon l'invention contre l'ulcère gastrique et son effet sur la diminution des taux d'histamine au niveau de la muqueuse gastrique, telles que démontrées dans la présente demande.

D'autres formes de réalisation de la composition suivant l'invention pour le traitement préventif et/ou curatif de l'ulcère peptique et des allergies gastro-intestinales sont indiquées dans les revendications annexées.

D'autres caractéristiques et avantages de l'invention ressortiront de la description donnée ci-après, à titre non-limitatif et en faisant référence aux exemples (et en particulier aux exemples comparatifs) décrits ci-dessous.

La figure 1 illustre les spectres RMN du complexe de (+)-catéchine monomérique et de lysine pour un ratio d'équivalence molaire 1 : 2 (a) et un ratio d'équivalence molaire 1 : 1 (b).

La figure 2 illustre les spectres RMN du complexe de (+)-catéchine monomérique et d'arginine pour un ratio d'équivalence molaire 1 : 2 (a) et un ratio d'équivalence molaire 1 : 1 (b).

La figure 3 illustre l'évolution des concentrations plasmatiques (cc) en (+)-catéchine libre mesurée chez l'être humain en ng/ml au cours du temps T (heure) après l'assimilation de chlorhydrolysinate de (+)-catéchine (riche en (+)-catéchine monomérique à hauteur de 61 % en poids, groupe A) et d'un gramme de (+)-catéchine monomérique pure (groupe B).

La figure 4 illustre le spectre de diffraction aux Rayons X (poudre) du complexe de (+)-catéchine monomérique et de lysine pour un ratio d'équivalence molaire 1 : 2.

La figure 5 illustre l'évolution de la concentration en (+)-catéchine monomérique libre dans le plasma en fonction du ratio d'équivalence molaire entre la (+)-catéchine monomérique et la Lysine : 1 : 0 ; 1 : 1, 1 : 2, 1 : 2,5, 1 : 3, et 1 : 5, pour un sel de complexe [C : Lys : HCl] (voir tableau 14).

Dans la description, les exemples 1 à 6 ainsi que les exemples comparatifs 1 à 5 portent sur les résultats de biodisponibilité *per* os et d'activité anticancéreuse obtenus chez le mammifère (rat et être humain) pour une composition à base d'un complexe [C :AA/1 : 1 - 1 : 2,5].

Les exemples comparatifs 6 à 10 décrivent les résultats de biodisponibilité *per* os obtenus chez le rat pour la composition à base du mélange de (+)-catéchine monomérique et d'au moins un acide aminé basique ou d'au moins un dérivé d'un acide aminé basique dans un ratio d'équivalence molaire compris entre 1 : 1 et 1 : 2,5, de préférence entre 1 : 1 et 1 : 2.

Dans le cadre de la présente invention, il a en outre été démontré l'équivalence entre les résultats des tests obtenus chez le rat et chez l'être humain. Il est donc prouvé que les résultats obtenus chez le rat sont effectivement reproduits chez l'être humain.

Dans les exemples qui suivent, à l'exception des résultats du tableau 7b, les teneurs en (+)-catéchines dans le sang correspondent aux teneurs en (+)-catéchine libre monomérique.

*Matériel et méthode : mesure de la biodisponibilité*

La biodisponibilité correspond à la proportion de (+)-catéchine monomérique libre qui se retrouve dans l'organisme par rapport à la quantité initialement administrée.

Dans le cadre de la présente invention, et à titre non limitatif, la (+)-catéchine peut être absorbée sous forme du complexe [C : AA] ou sous la forme de (+)-catéchine monomérique pure, c'est-à-dire qui n'est pas associée avec au moins un acide aminé basique.

Dans le cadre de la présente invention, il y a tout d'abord lieu de distinguer, la (+)-catéchine ayant été absorbée, la présence dans le sang de (+)-catéchine monomérique libre, c'est-à-dire sous une forme non-conjuguée, de la (+)-catéchine conjuguée, c'est-à-dire qui a été dérivatisée par le métabolisme du mammifère.

La (+)-catéchine monomérique dérivatisée résulte du cycle d'élimination de la (+)-catéchine monomérique libre dans le sang par le métabolisme (par exemple et de manière non limitative, par le cycle entéro-hépatique).

L'élimination de substances (ou molécules) actives étrangères à l'organisme résulte de l'action conjointe de plusieurs processus. Elle comprend la capacité métabolique de différents organes, en premier lieu du foie, et l'excrétion sous toutes ses formes, en particulier rénale (urine) mais aussi hépatique (bile).

Ce métabolisme d'élimination prévoit la dérivation de la (+)-catéchine monomérique par un cycle de biotransformation bien connu qui implique 2 phases de métabolisme selon les processus de transformation enzymatique. On distingue donc les réactions de phase I et les réactions de phase II :

- les réactions de phase I comprennent les réactions d'oxydation qui sont majoritairement localisées dans les microsomes hépatiques ; les réactions de réduction qui sont beaucoup moins fréquentes et moins bien explorées ; et les réactions d'hydrolyse qui constituent une voie métabolique banale, qui intervient dans le foie, dans différents tissus et même dans le plasma ; et ensuite

- les dérivés issus des réactions de phase I sont ensuite conjugués. C'est cette conjugaison qui constitue les réactions de phase II, y compris la glucuro-conjugaison qui fait intervenir la conjugaison de ces dérivés avec l'acide glucuronique.

Dans ce contexte, à une proportion (ou une teneur) déterminée de (+)-catéchine monomérique libre dans le sang après administration *per* os de (+)-catéchine monomérique, correspond une biodisponibilité déterminée.

**- Mode opératoire** :

Les résultats expérimentaux décrits ci-dessous ont été obtenus sur base d'une étude réalisée chez l'être humain sain et chez le rat Wistar sain pesant approximativement 250 grammes.

En ce qui concerne les études menées sur le rat, les individus ont été logés par groupe de 4 individus maximum dans une cage dans une litière de sciure à une température ambiante comprise entre 20°C et 25°C, avec un éclairage de 12 h sur 24 h. Une période d'acclimatation a été respectée avant d'entamer les expériences.

Durant l'étude expérimentale, les rats ont eu accès à une nourriture standard commerciale et à de l'eau *ad libitum.* Les rats ont été mis au jeûne toute la nuit qui précède l'administration de la source en produit actif, étant entendu que le produit actif est de la (+)-catéchine monomérique (ou des autres polyphénols : la quercétine et l'EGCG).

Les rats ont reçu une administration *per* os d'un volume de 5 ml d'une solution en produit actif.

En ce qui concerne l'échantillonnage des prélèvements sanguins chez le rat et chez l'être humain, le sang a été récolté dans des tubes à revêtement interne à l'EDTA pour être ensuite centrifugé à 3000 rpm (rotation par minute) à température ambiante pendant 45 min, et ensuite durant 15 min à une température de 4°C. le plasma a ensuite été collecté et stocké à -70°C avant analyse.

La technique analytique (LC-MS/MS pour *Liquid Chromatography coupled to tandem Mass Spectrometry)* pour la mesure en substance active dans le sang s'inspire de la méthode décrite par Mata-Bilbao et co. Publiée an 2007 dans le Journal of Agricultural and Food Chemistry, volume 55, page 8857.

En ce qui concerne le développement méthodologique, les plasmas de référence ont été collectés en interne, à partir de rats et de vaches non traitées par la substance active, et ensuite stockés à -20°C.

La (+)-catéchine libre a été extraite du plasma avec une solution d'acide phosphorique, d'EDTA et d'acide ascorbique pour être ensuite purifiée par SPE (pour *solid phase extraction*) en particulier, il s'agit d'une extraction *Oasis™ HLB* et analysée par LC-MS/MS. La quantification a été réalisée selon une procédure de calibration standard avec comme standard interne (SI) la (+/-)-catéchine-2,3,4-$^{13}$C3.

Dans ce contexte, la teneur en (+)-catéchine libre reprend non seulement la teneur en (+)-catéchine libre monomérique isolée du plasma, mais aussi ses formes isomères qui résultent de l'isomérisation de la (+)-catéchine libre monomérique afférente aux conditions d'extraction de la (+)-catéchine libre dudit plasma.

La procédure est identique en ce qui concerne l'extraction et la quantification de la quercétine ou de l'ECGC libre dans le sang.

En ce qui concerne l'extraction et la quantification de la (+)-catéchine totale, le mode opératoire est le même que celui appliqué pour la (+)-catéchine libre, à l'exception du fait qu'une étape supplémentaire et préalable à la purification par SPE est réalisée. Cette étape supplémentaire consiste à traiter l'échantillon avec une solution digestive d'arylsulfatase et de glucoronidase, afin d'extraire la substance active des cellules plasmatiques.

La concentration totale (ct) en (+)-catéchine (libre ou dérivatisée) est calculée en mesurant, par exemple par la méthode des trapèzes, l'aire sous la courbe d'évolution des concentrations plasmatiques (cc) mesurées en ng/ml au cours du temps après administration orale de la source en (+)-catéchine monomérique.

**- Standards**

Une solution de standard interne de 1 mg/ml (méthanol) de (+)-catéchine a été préparée à partir de (+)-catéchine de stock pour ensuite être stockée à -20°C.

La catéchine-C13 (référence: 719579, de pureté de 99,3% en poids) produite par *Sigma Aldrich,* a été utilisée comme SI.

La solution de SI pour la quantification de la (+)-catéchine (libre et totale) a été préparée en diluant 5 ml de la solution de stock dans du méthanol dans un volume final de 10 ml. Cette solution de Si a ensuite été stockée à -20°C.

**- Méthodes**

*a) Protocole d'extraction pour l'analyse de la (+)-catéchine monomérique libre dans le plasma*

- un transfert de 500 µl de plasma homogénéisé dans un flacon de 15 ml ;
- un ajout de 50 µl de SI (10 ppm) ;
- un ajout de 180 µl d'une solution antioxydante (20 mg/ml d'acide ascorbique et 1 mg/ml d'EDTA) et 10 µl d'acide ortho-phosphorique ;
- un mélange vortex pendant 2 min ;
- un ajout de 1,5 ml d'eau pour dilution pour obtenir un mélange d'extraction ;

- une extraction appliquée au mélange d'extraction par phase solide sous *Water Oasis™ HLB* selon le protocole standard bien connu de l'homme de l'art et adaptable à la présente procédure ;
- un séchage de l'éluat par évaporation à 45°C sous atmosphère inerte ;
- une solubilisation de l'extrait sec dans de l'acétonirile, suivie d'une centrifugation à 3000 rpm pendant 10 min; et
- une analyse HPLC (pour *high performance liquid chromatography).*

*b) Protocole d'extraction pour l'analyse de la (+)-catéchine monomérique totale dans le plasma*

- un transfert de 500 μl de plasma homogénéisé dans un flacon de 15 ml ;
- un ajout de 50 μl de SI (10 ppm) ;
- un ajout de 180 μl d'une solution antioxydante (20 mg/ml d'acide ascorbique et 1 mg/ml d'EDTA) et 10 μl d'acide ortho-phosphorique ;
- un mélange vortex pendant 2 min ;
- un ajout de 750 μl d'une solution tampon d'acétate 0,2 M (pH de 4,8) ;
- un ajout de 5 μl d'un mélange d'*Helix pomatia* qui suit une étape de digestion de la solution pendant 2 heures à 55°C ;
- une centrifugation à 4000 rpm pendant 10 min pour obtenir un mélange d'extraction ;
- une extraction appliquée au mélange d'extraction par phase solide sous *Water Oasis™ HLB* selon le protocole standard bien connu de l'homme de l'art et adaptable à la présente procédure ;
- un séchage de l'éluat par évaporation à 45°C sous atmosphère inerte ;
- une solubilisation de l'extrait sec dans de l'acétonirile, suivie d'une centrifugation à 3000 rpm pendant 10 min; et
- une analyse HPLC (pour *high performance liquid chromatography).*

*c) Extraction - Water Oasis™ HLB*

- Solution d'activation :

  ○ 1 ml de méthanol ;
  ○ 1 ml d'eau ; et
  ○ 1 ml d'une solution de DMF (70% en volume) contenant 0,1% (en volume) d'acide formique.

- Solution de lavage :

  ○ E ml d'eau ;
  ○ 1 ml d'une solution de méthanol (30% en volume) ;
  ○ 1 ml d'acétate d'éthyle.

- Solution d'élution
  ○ 5 ml d'un mélange d'acétate d'éthyle et de méthanol dans un ratio d'équivalence molaire de 1 : 2.

*d) Analyse LC-MS/MS*

• **Chromatographie liquide (LC)**

- colonne : *Alltima C18 5u ;*
- phase mobile aqueuse A HPLC : acide formique (concentrée à hauteur de 0,1% en volume) ;
- phase mobile B HPLC : solution d'acétonitrile contenant de l'acide formique à hauteur de 0,1 % en volume ;
- vitesse de flux : 0,6 ml/min ;
- température de four : 30 °C ; et
- volume d'injection : 50 μl ;
- programme d'élution :

| Temps (min) | A (% volumique) | B (% volumique) |
|---|---|---|
| 0,00 | 95 | 5 |
| 0,50 | 95 | 5 |
| 4,00 | 100 | 0 |
| 4,50 | 100 | 0 |
| 5,50 | 95 | 5 |
| 8,00 | 95 | 5 |

**· Spectroscopie de masse (MS)**

- ionisation ESI en mode négatif avec une température de désolvatation de 400°C ;
- température de source : 120°C ;
- flux du gaz de cône : 150 l/h ;
- flux de gaz de désolvatation : 1000 l/h.

*Synthèse d'un complexe*

Le complexe correspond à un édifice moléculaire dans lequel la (+)-catéchine monomérique est liée audit au moins un acide aminé, ou audit au moins un dérivés d'acide aminé basique, par des liaisons de type pont hydrogène.

Ceci signifie que, lorsque le complexe ne se dissocie pas en solution aqueuse.

La (+)-catéchine monomérique correspond à un polyphénol présent sous la forme d'un monomère.

L'acide aminé basique présente un radical qui est chargé positivement à pH neutre.

Le dérivé d'acide aminé basique quant à lui est défini comme une molécule provenant d'un acide aminé basique, et qui résulte d'une ou plusieurs transformations chimiques opérées sur cet acide aminé.

De manière préférentielle, le complexe (sous forme de sel ou non) comprend de la (+)-catéchine monomérique et au moins un précurseur d'acide aminé qui est une molécule destinée à être transformée en acide aminé ou en dérivé d'acide aminé après administration orale.

Par exemple, et à titre non-limitatif, le précurseur d'acide aminé peut être un glucuronide ou un glucuronoside d'acide aminé, qui, une fois absorbé, est transformé par l'organisme en acide aminé, de par l'action des enzymes β-glucuronidases.

**Exemple 1** : **préparation d'un complexe de (+)-catéchine monomérique et de lysine**

1a. Complexe de (+)-catéchine *monomérique* et de lysine dans un ratio d'équivalence molaire 1 : 2

De la (+)-catéchine monomérique, extraite de *l'Uncaria Gambir* selon un des procédés bien connus de l'homme de l'art, est broyée puis séchée à 50°C sous vide pendant 30 minutes.

Après cette étape de séchage, la (+)-catéchine extraite comprend de l'eau au moins en traces.

Deux équivalents de lysine sont ensuite ajoutés successivement à la (+)-catéchine sous agitation jusqu'à dissolution dans de l'eau distillée dans laquelle de l'hélium y est bullé. La solution est ensuite chauffée à une température comprise entre 40°C et 45°C et agitée durant environ une heure jusqu'à dissolution complète des deux équivalents de lysine et est ensuite mise au frigo 16h.

De manière alternative, la (+)-catéchine peut être ajoutée à une solution de deux équivalents de lysine amenée à une température comprise entre 40°C à 45°C sous agitation.

La solution est ensuite filtrée sur buchner et lavée avec 50ml d'eau distillée pour être ensuite séchée avec évaporation de l'eau d'abord au rotavapor et ensuite à la rampe à vide (pendant 4h à une température de 50°C).

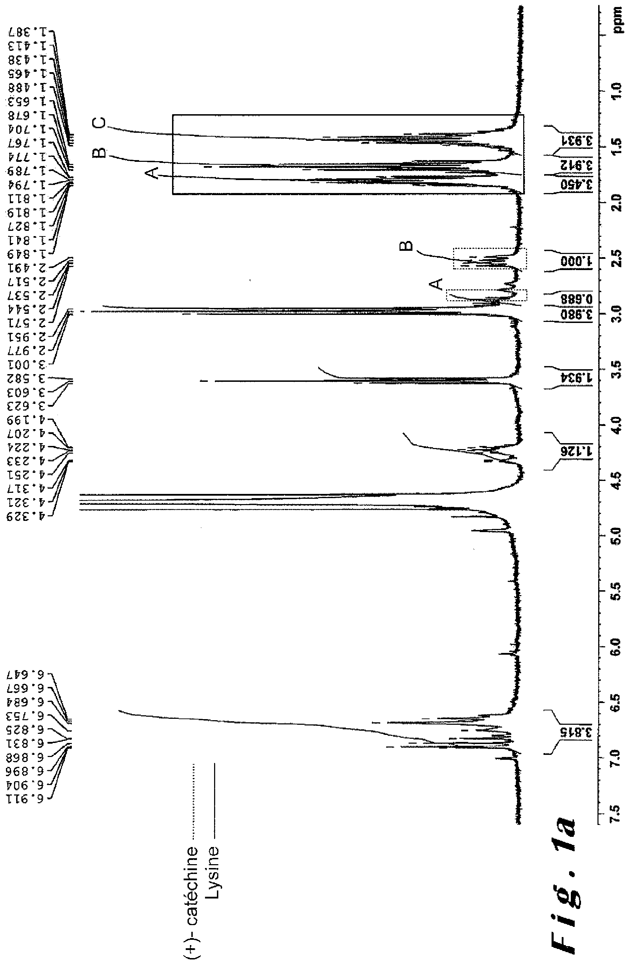

Le produit obtenu n'est pas soluble dans le méthanol deutéré mais dans l'eau deutérée ($D_2O$, solvant utilisé pour l'analyse RMN). Le spectre RMN obtenu est illustré en figure 1a.

A la lecture du spectre RMN, on observe que le complexe est formé de deux moles de lysine pour une mole de (+)-catéchine monomérique.

Par ailleurs, la présence des trois pics A, B et C sur le spectre montre que la lysine est bien liée à la catéchine. On observe en effet une forte modification des pics aromatiques de la(+)-catéchine avec une disparition des pics vers 5,95-5,87 ppm et une mauvaise intégration des pics au niveau de 6,50-7,00 ppm. Cela indique qu'il y a une interaction entre la lysine et les cycles aromatiques de la (+)-catéchine, au niveau des groupements phénoliques, ces interactions impliquant des liaisons de type pont hydrogène.

Des spectres de diffraction aux Rayons X collectés en utilisant un diffractomètre à poudre *Siemens D5000* avec une radiation Cu Kα (À = 1,542 Å) ont été pris pour les composés suivants : la (+)-catéchine monomérique (JE143), un mélange (JECatLysH) d'hydrochlorure de lysine, de (+)-catéchine monomérique, et de lysine protonée (mélange C : Lys : HCl/ 1 : 2 :2), et trois mélanges de (+)-catéchine monomérique et de lysine non-protonée (JE149b, JE150, JE151). Les données ont été enregistrées dans une plage comprise entre 5° et 60° par pas de 0,0167°.

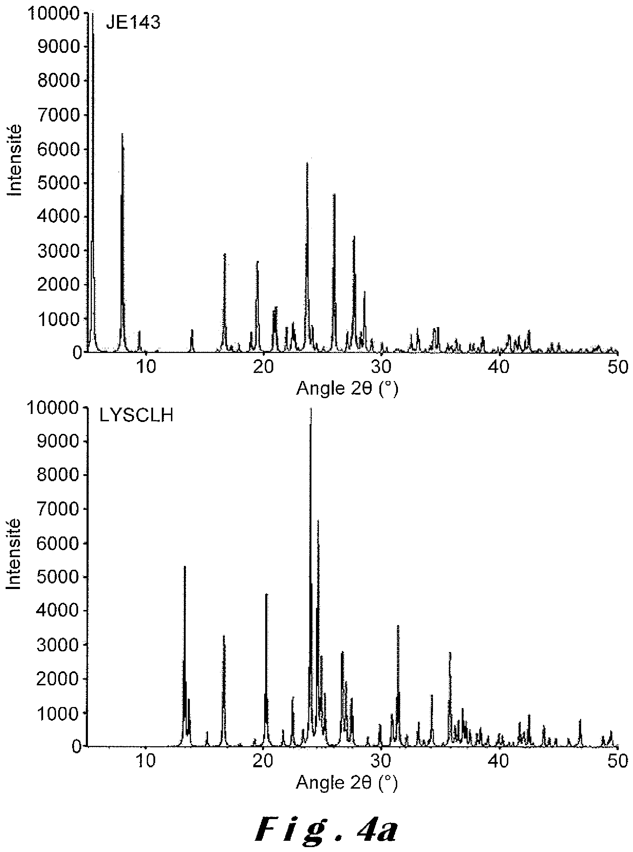

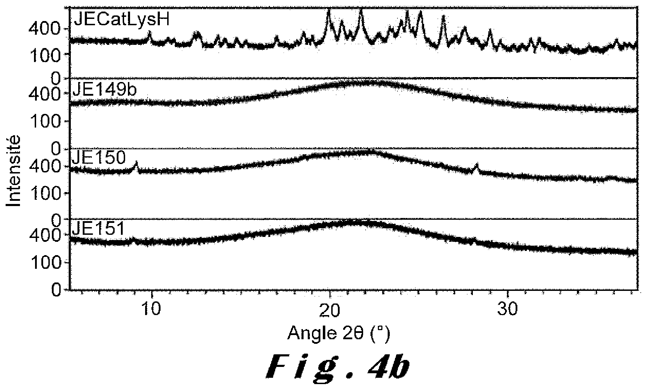

Les spectres sont illustrés à la figure 4. Comme le montre la figure 4a, à l'échantillon JECatLysH est associé un spectre DRX qui n'apparaît pas être un recouvrement des spectres de la (+)-catéchine monomérique et de l'hydrochlorure de lysine , montrant ainsi la formation d'un complexe et non d'un mélange des deux composants. En particulier on note entre autres la disparition des pics aux angles de 31,5 et 36 degrés de la lysine dans le spectre du complexe

Les spectres pour les trois mélange de (+)-catéchine monomérique et de lysine non-protonée (JE149b, JE150, JE151) ne montrent aucun indice de cristallinité pour ces compositions, ce qui indique que, pour ces références JE149b, JE150, JE151, il y a formation d'une composition à l'état cristallin amorphe, contrairement à ce qui est observé pour le mélange JECatLysH pour lequel il a été identifié un réseau cristallin qui ne correspond pas à la superposition des spectres de l'hydrochlorure de lysine et de la (+)-catéchine monomérique.

1b. Complexe de (+)-catéchine *monomérique* et de lysine dans un ratio d'équivalence molaire 1 : 1

Le complexe de (+)-catéchine monomérique avec la lysine dans un ratio d'équivalence molaire 1 : 1 a été synthétisé selon le même protocole que celui employé pour la synthèse du complexe de lysine de l'exemple 1a mais en diminuant par un facteur 2 la quantité de lysine de telle sorte qu'il existe un excès de (+)-catéchine qui favorise la formation dudit complexe sous un ratio d'équivalence molaire 1 : 1 de par le déficit en lysine.

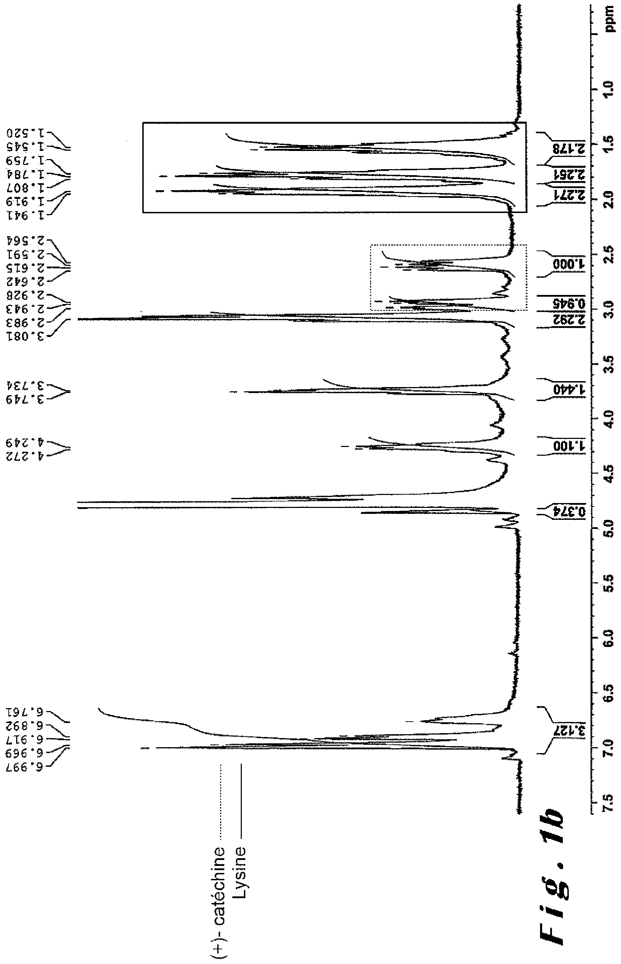

Le spectre RMN tiré dans l'eau deutérée est illustré en figure 1b. A la lecture du spectre RMN, on observe que le complexe est formé d'une mole de lysine pour une mole de (+)-catéchine monomérique

**Exemple 2 : préparation d'un complexe de (+)-catéchine monomérique et d'arginine**

2a. Complexe de (+)-catéchine monomérique et d'arginine dans un ratio d'équivalence molaire 1 : 2

Le complexe de (+)-catéchine monomérique avec l'arginine a été synthétisé selon le même protocole que celui employé pour la synthèse du complexe de lysine de l'exemple 1a.

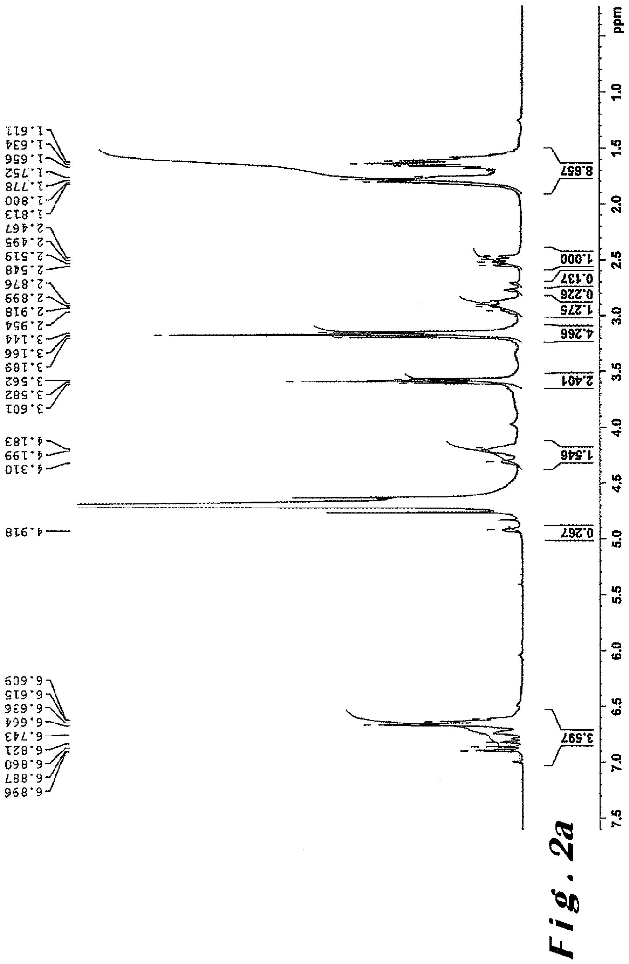

A la lecture du spectre RMN illustré en figure 2a, on observe que le complexe est formé de deux molécules d'arginine liées par des liaisons de type pont hydrogène à une molécule de (+)-catéchine monomérique.

2b. Complexe de (+)-catéchine monomérique et d'arginine dans un ratio d'équivalence molaire 1 : 1

Le complexe de (+)-catéchine monomérique avec l'arginine a été synthétisé selon le même protocole que celui employé pour la synthèse du complexe de lysine de l'exemple 1b.

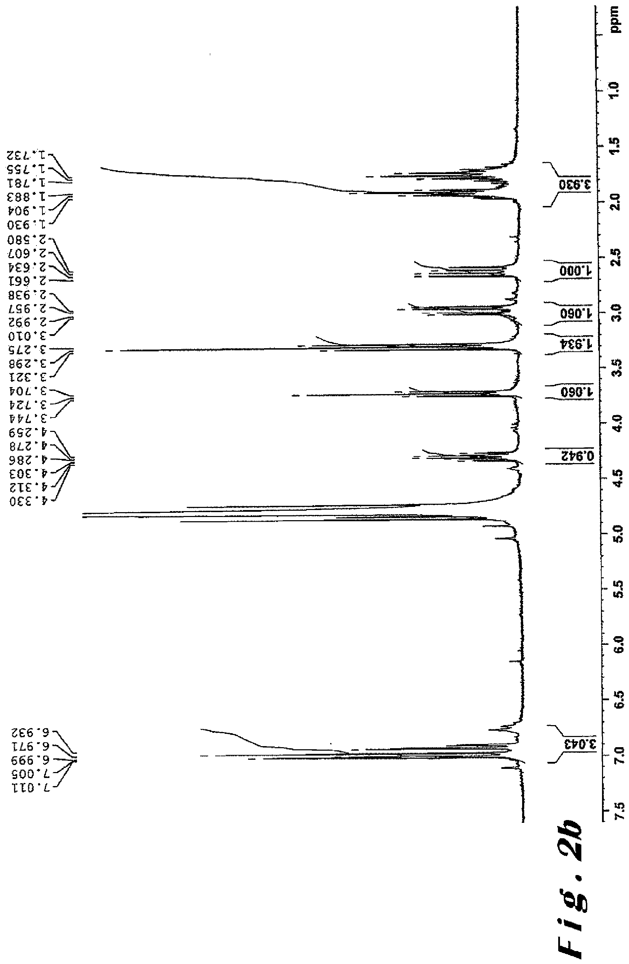

A la lecture du spectre RMN dudit complexe illustré à la figure 2b, on observe que le complexe est formé d'une molécule d'arginine liée par des liaisons de type pont hydrogène à une molécule de (+)-catéchine monomérique.

*Biodisponibilité par voie orale du complexe*

**Exemple 3 : mesure de la biodisponibilité par voie orale du chlorhydrolysinate de (+)-catéchine monomérique (ou sel de complexe [C : Lys : HCl] chez l'être humain**

1,5 g d'un complexe de (+)-catéchine monomérique et d'au moins un acide aminé basique : le chlorhydrolysinate de (+)-catéchine monomérique. A partir du complexe [C : Lys] obtenu selon le protocole 1b, la (+)-catéchine monomérique est présente à raison de 61% en masse par rapport au poids total dudit complexe (soit 0,92 g pour 1,5 g), le chlorhydrolysinate de (+)-catéchine monomérique (sel de complexe [C : Lys : HCl]) est synthétisé par neutralisation dudit complexe [C : Lys] par de l'HCl ajouté en quantité équimolaire à celle dudit complexe.

De cette façon, un sel de complexe dans lequel la lysine est liée au proton (Lys-H$^+$) issu du HCl et dans lequel le chlorure (anion Cl$^-$) est libre en solution et n'interagit pas avec le complexe [C : Lys-H$^+$].

Ce sel de complexe est administré à un groupe A de 5 volontaires sains.

La teneur en (+)-catéchine monomérique libre dans le sang a été mesurée pour le groupe A de volontaires au cours du temps après la prise de 1,5 g chlorhydrolysinate de (+)-catéchine monomérique contenant 0,92 g de (+)-catéchine monomérique (tableau 1).

Tableau 1: concentration plasmatique (cc) de la (+)-catéchine monomérique libre (en ng/ml) pour le groupe A en fonction du temps T (heure)

| | Groupe |
|---|---|
| T (h) | A |
| | cc (ng/ml) |
| 0,5 | 524 ± 30 |
| 1 | 487 ± 47 |
| 2 | 328 ± 35 |
| 3 | 173 ± 38 |
| 4 | 78 ± 24 |
| 6 | 16 ± 2 |

Tableau 2: paramètres d'évaluation de la biodisponibilité par voie orale calculés à partir des courbes de concentrations plasmatiques (cc) de la (+)-catéchine monomérique libre (en ng/ml) pour le groupe A en fonction du temps T (heure)

| | Groupe |
|---|---|
| | A |
| T (max) (h) | 0,5 |
| ct (ng h/ml) (ng min/ml) | 1263 ± 88 (75780) |
| c (max) (ng/ml) | 525 |

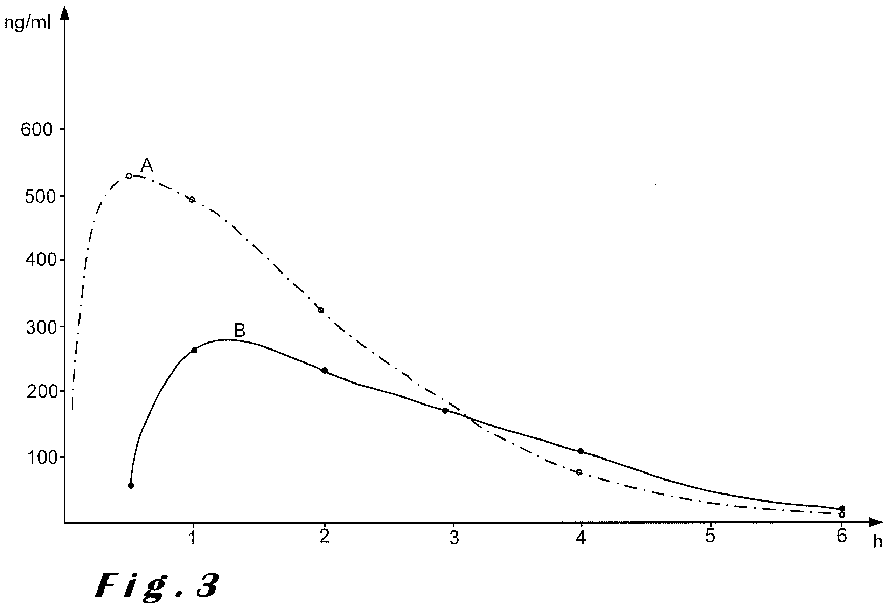

La figure 3 illustre l'évolution des concentrations plasmatiques (cc) mesurées en ng/ml au cours du temps T (heure) après administration orale de chlorhydrolysinate de (+)-catéchine (courbe A), cette courbe est extrapolée à partir des données répertoriées dans le tableau 1.

L'aire mesurée, par exemple par la méthode des trapèzes bien connue de la littérature, sous la courbe indique la concentration totale (ct) de (+)-catéchine monomérique libre assimilée dans le plasma mesurée sur une durée de six heures à partir de l'ingestion de la (+)-catéchine sous forme d'un complexe formé avec le chlorhydrolysinate. Ce paramètre ct permet de calculer, à partir des données du tableau 1, la biodisponibilité de la (+)-catéchine exprimée en ng h/ml. La ct est de 1263 ± 88 ng h/ml pour le chlorhydrolysinate de (+)-catéchine.

De la figure 3, la concentration maximale c (max) correspond au pic de la courbe. Pour le groupe ayant reçu le sel de complexe [C : Lys : HCl], le c (max) atteint 525 ng/ml. Cette concentration maximale est atteinte à un temps T (max) de 0,5 h pour le groupe A ayant reçu le complexe de (+)-catéchine monomérique.

**Exemple comparatif 1** : **mesure de la biodisponibilité par voie orale de la (+)-catéchine monomérique pure chez l'être humain**

Une dose de 1 g de (+)-catéchine monomérique pure est administrée à un groupe B de 5 volontaires sains.

La teneur en (+)-catéchine monomérique libre dans le sang a été mesurée pour le groupe de volontaires B au cours du temps après la prise de (+)-catéchine. Les résultats sont indiqués au tableau 3.

Tableau 3: concentration plasmatique (cc) de la (+)-catéchine monomérique libre (en ng/ml) pour le groupe B en fonction du temps T (heure)

| | Groupe |
|---|---|
| T (h) | B |
| | cc (ng/ml) |
| 0,5 | 59 ± 61 |
| 1 | 266 ± 147 |
| 2 | 230 ± 87 |
| 3 | 226 ± 38 |
| 4 | 128 ± 51 |

EP 2 996 686 B1

(suite)

| T (h) | Groupe |
|---|---|
| | B |
| | cc (ng/ml) |
| 6 | 27 $\pm$ 13 |

Tableau 4: paramètres d'évaluation de la biodisponibilité par voie orale calculés à partir des courbes de concentrations plasmatiques (cc) de la (+)-catéchine monomérique libre (en ng/ml) pour le groupe B en fonction du temps T (heure)

| | Groupe |
|---|---|
| | B |
| T (max) (h) | 1,20 |
| ct (ng h/ml) (ng min/ml) | 904 $\pm$ 163 (54240) |
| c (max) (ng/ml) | 280 |

La figure 3 illustre l'évolution des concentrations plasmatiques (cc) mesurées en ng/ml au cours du temps T (heure) après administration orale de (+)-catéchine pure (courbe B). Cette courbe est extrapolée à partir des données répertoriées dans le tableau 3.

L'aire mesurée sous la courbe indique la concentration totale (ct) de (+)-catéchine monomérique libre assimilée dans le plasma mesurée sur une durée de six heures à partir de l'ingestion de la (+)-catéchine pure. Ce paramètre ct permet de calculer, à partir des données des tableaux 1 et 3, la biodisponibilité de la (+)-catéchine exprimée en ng h/ml. La ct est calculé à 1263 $\pm$ 88 ng h/ml pour 1,5g de chlorhydrolysinate de (+)-catéchine contenant 0,92g de (+)-catéchine pure. Il est calculé à 904 $\pm$ 163 ng h/ml pour 1g de (+)-catéchine pure, soit à 832 $\pm$ 150 ng h/ml pour 0,92g de (+)-catéchine pure, correspondant à la dose ingérée avec le sel de complexe [C:Lys :HCl] (voir exemple 2), soit une amélioration de la biodisponibilité d'au moins 52%.

Dans la figure 3, les concentrations maximales c (max) correspondent aux pics des courbes. Pour le groupe ayant reçu 1g de (+)-catéchine monomérique pure, le c (max) est calculé à 280 ng/ml, soit 258 ng/ml pour 0,92g de (+)-catéchine pure correspondant à la dose de (+)-catéchine ingérée avec le sel de complexe [C:Lys :HCl]. Pour le groupe ayant reçu le sel de complexe [C : Lys : HCl], le c (max) atteint 525 ng/ml, ce qui correspond à une augmentation de 103% par rapport à la (+)-catéchine pure. Ces concentrations maximales son atteintes respectivement à un temps T (max) de 1,20 h pour le groupe B et de 0,5 h pour le groupe ayant été traité par le complexe de (+)-catéchine monomérique.

De ce premier exemple comparatif, on montre que le complexe de (+)-catéchine, et en particulier le chlorhydrolysinate de (+)-catéchine améliore de façon significative (+52%) la biodisponibilité par voie orale de la (+)-catéchine monomérique par rapport à sa prise sous forme pure. La rapidité de passage de la (+)-catéchine monomérique dans le sang (et présente ensuite sous forme libre dans le plasma), mesuré par le paramètre T (max), ainsi que la concentration maximale, c (max), sont également très significativement augmentés : respectivement de +166% et de +103%, par rapport à la prise de (/+)-catéchine monomérique sous sa forme pure.

**Exemple comparatif 2** : **mesure de la biodisponibilité par voie orale de la (+)-catéchine monomérique pure et du complexe de (+)-catéchine et de lysine chez le rat**

Une dose de 100 mg (par kg de poids corporel) de (+)-catéchine monomérique pure (CP) ou une dose de lysinate de (+)-catéchine équivalente à 100 mg de (+)-catéchine a été administrée chez chaque individu d'un échantillon de 5 rats Wistar.

Les paramètres d'évaluation de la biodisponibilité par voie orale sont donnés au tableau 5 pour différentes sources de (+)-catéchine : CP, ou différents complexes de lysine.

Tableau 5: paramètres d'évaluation de la biodisponibilité par voie orale calculés à partir des courbes de concentrations plasmatiques (cc) de la (+)-catéchine monomérique libre (en ng/ml) pour chaque source de (+)-catéchine en fonction du temps T (minute)

| | Source de (+)-catéchine monomérique | | | |
|---|---|---|---|---|
| | CP | [C : Lys/1 : 1] | [C: Lys : HCL/1 : 1 : 1] | [C : Lys/1 : 2] |
| T (max) (min) | 60 | 60 | 60 | **120** |
| c (max) (ng/ml) | 716 | 1127 | 955 | **1547** |
| ct (ng min/ml) | 96238 | 112422 | 132349 | **186348** |
| Δct (en %) | 0 | +17 | +36 | **+94** |

Dans ce tableau, le Δct (exprimé en %) correspond à la mesure de la différence entre la ct obtenu avec chacun des complexe et la ct mesurée pour la (+)-catéchine monomérique pure, ramenée à la valeur de biodisponibilité pour la CP. A titre illustratif, le Δct calculé pour le complexe [C : Lys /1 : 1] est obtenu comme suit :

$$100 \times [(112422 - 96238)/ 96238] = 17 \%$$

Les meilleures valeurs de paramètre sont atteintes pour le complexe [C : Lys /1 : 2] pour lequel une valeur de biodisponibilité par voie orale de près de $1,9 \times 10^5$ ng min/ml est atteinte, ce qui correspond à une augmentation de 94% par rapport à la (+)-catéchine ingérée sous forme pure.

De plus la concentration maximale [C(max)] de catéchine passée dans le sang est augmentée de 116%, le pic donnant cette Cmax est situé à 120 min [T (max)] contre 60 min pour les autres sources, ce qui a également pour effet d'augmenter favorablement les taux sanguins en (+)-catéchine libre monomérique dans le temps, confirmant l'action différée de la substance active et donc une action anticancéreuse améliorée.

Ces données correspondent à ce que nous avions trouvé chez l'être humain en comparant la prise orale de (+)-catéchine pure (tableau 4) à celle du sel de complexe [C :Lys :HCL, 1 : 1 : 1] (tableau 2). Les biodisponibilités sont en effet augmentées dans les mêmes proportions, +52% chez l'homme, + 36% chez le rat.

Dans le présent exemple, chez le rat, nous montrons que le complexe [C :Lys/1: 2] augmente encore plus significativement la biodisponibilité de la (+)-catéchine par voie orale.

**Exemple comparatif 3 : mesure de la biodisponibilité par voie orale de la (+)-catéchine monomérique pure, du complexe de (+)-catéchine et d'arginine, et du complexe de (+)-catéchine et de lysine, chez le rat**

Une dose de 100 mg (par kg de poids corporel) de (+)-catéchine monomérique pure (CP) ou une dose de complexe de (+)-catéchine équivalente à 100 mg de (+)-catéchine a été administrée chez chaque individu d'un échantillon de 5 rats Wistar.

Les paramètres d'évaluation de la biodisponibilité par voie orale sont donnés au tableau 6 pour différentes sources de (+)-catéchine : CP, ou différents complexes de lysine ou d'arginine.

Tableau 6: paramètres d'évaluation de la biodisponibilité par voie orale calculés à partir des courbes de concentrations plasmatiques (cc) de la (+)-catéchine monomérique libre (en ng/ml) pour chaque source de (+)-catéchine en fonction du temps T (minute)

| | Source de (+)-catéchine monomérique | | |
|---|---|---|---|
| | CP | [C : Lys /1 : 1] | [C : Arg /1 : 1] |
| T (max) (min) | 60 | 60 | 60 |
| c (max) (ng/ml) | 716 | 1127 | 1292 |
| ct (ng min/ml) | 96238 | 112422 | 116351 |
| Δct (en %) | 0 | +17 | +21 |

De ces résultats, il est déduit que tout acide aminé basique (c'est-à-dire choisi parmi la lysine, l'arginine, et l'histidine) se liant à la (+)-catéchine, et donc neutralisant l'acidité des groupements ortho-diphénols situés sur le noyau de la (+)-catéchine, pourraient avoir le même effet bénéfique sur la biodisponibilité par voie orale de celle-ci.

**Exemple comparatif 4 : mesure de la biodisponibilité par voie orale de la (+)-catéchine monomérique pure, du complexe de (+)-catéchine et de lysine, à un ratio d'équivalence molaire de 1 : 1 et 1 : 5, chez le rat**

Une dose de 25 mg (par kg de poids corporel) de (+)-catéchine monomérique pure (CP) ou une dose de lysinate de (+)-catéchine équivalente à 25 mg de (+)-catéchine a été administrée chez chaque individu d'un échantillon de 3 rats Wistar.

Les paramètres d'évaluation de la biodisponibilité par voie orale sont donnés au tableau 7a pour différentes sources de (+)-catéchine : CP, ou différents complexes de lysine.

Les paramètres de T (max), c (max) et de ct en (+)-catéchine totale présente dans le sang sont donnés au tableau 7b. La (+)-catéchine totale reprend la (+)-catéchine monomérique libre et la (+)-catéchine monomérique qui a été conjuguée par l'organisme du rat.

Tableau 7a : paramètres d'évaluation de la biodisponibilité par voie orale calculés à partir des courbes de concentrations plasmatiques (cc) de la (+)-catéchine monomérique libre (en ng/ml) pour chaque source de (+)-catéchine en fonction du temps T (minute)

| | Source de (+)-catéchine monomérique | | |
|---|---|---|---|
| | CP | [C : Lys /1 : 2] | [C : Lys /1 : 5] |
| T (max) (min) | 30 | 60 | 60 |
| c (max) (ng/ml) | 154 | 173 | 74 |
| ct (ng min/ml) | 21145 | 24845 | 13770 |
| $\Delta$ct (en %) | 0 | +17 | -35 |

Tableau 7b: paramètres de T (max), c (max) et de ct en (+)-catéchine totale présente dans le sang calculés à partir des courbes de concentrations plasmatiques (cc) de la (+)-catéchine *monomérique* totale (en ng/ml) pour chaque source de (+)-catéchine en fonction du temps T(minute)

| | Source de (+)-catéchine monomérique | | |
|---|---|---|---|
| | CP | [C : Lys /1 : 2] | [C : Lys /1 : 5] |
| T (max) (min) | 30 | 60 | 60 |
| c (max) (ng/ml) | 2200 | 2366 | 1100 |
| ct (ng min/ml) | 273875 | 339245 | 163080 |
| $\Delta$ct (en %) | 0 | +24 | -40 |

Le complexe [C :Lys/ 1 : 2] permet une augmentation à la fois des teneurs en (+)-catéchine plasmatique libre et totale tandis que le complexe [C :Lys/ 1 : 5] diminue nettement ces teneurs par rapport à la (+)-catéchine pure, c'est-à-dire ingérée sans lysine.

Les résultats de cet exemple comparés aux résultats illustrés au tableau 5 montrent que l'optimum en termes de biodisponibilité par voie orale est effectivement atteint pour le complexe [C :AA/1 : 2], en présence ou non d'HCl. Il est à noter que la présence d'un acide influence favorablement les paramètres de biodisponibilité par voie orale (voir tableau 5), toutefois, la présence de cet acide n'est pas essentielle pour obtenir une biodisponibilité par voie orale augmentée.

*Action anticancéreuse*

**Exemple 4 : mesure de l'action du chlorhydrolysinate de (+)-catéchine monomérique et de l'ascorbolysinate de (+)-catéchine monomérique sur l'incorporation totale et dans le collagène de la 3H-proline au niveau de la peau**

La stabilisation des membranes des lysosomes empêche la libération des enzymes protéolytiques responsables de la dégradation de la matrice conjonctive, notamment du collagène. Cette dégradation est à l'origine de la propagation des cellules cancéreuses métastasiques quelques soit le type de cancer (Cell Communication and Signaling 2010, 8 : 22).

La peau est un tissu particulièrement riche en matrice conjonctive et en fibres de collagène, ce qui lui donne toute son élasticité.

L'efficacité du chlorhydrolysinate de (+)-catéchine et de l'ascorbolysinate de (+)-catéchine monomérique administrés par voie orale, d'une part sur l'incorporation totale (sur toute la peau) et d'autre part dans le collagène de celle-ci, de la 3H-proline est démontrée ici.

Pour ce faire, une étude a été menée *in vivo* sur un lot de 24 rats Wistar femelles pesant de 95 à 115 g, divisés en deux groupes C et D, chacun de 12 rats. Pour chaque groupe, la 3H-hydroxyproline radioactive, dont l'activité spécifique est de 13,6 Ci/$10^{-3}$ mol, a été administrée à l'ensemble des animaux par injection intrapéritonéale de proline-L-(5-3H) à une dose par rapport au poids des individus de 1 mCi/kg, ce qui représente une activité en 3H-proline radioactive de 100 μCi/individu.

6 rats de chaque groupe ont reçu un traitement à base de (+)-catéchine monomérique par gavage à raison de 100mg/kg pendant 5 jours avant le sacrifice. Les 6 autres rats n'ayant pas reçu de traitement à base de (+)-catéchine monomérique est un sous-groupe de contrôle (ctrl). Le groupe C a reçu du chlorhydrolysinate de (+)-catéchine monomérique tandis que le groupe D a reçu de l'ascorbolysinate de (+)-catéchine monomérique.

Tableau 8: action de la (+)-catéchine monomérique sous ses formes complexes (100 mg/kg) sur le taux (en dpm/mg) d'incorporation totale (IT) et dans le collagène (IC) de 3H-hydroxyproline dans la peau de rat

| Taux | Groupe | | | |
| | C | | D | |
| | ctrl | traité | ctrl | traité |
|---|---|---|---|---|
| IT | 5620 ± 186 | 6248 ± 124 | 4864 ± 231 | 5112 + 259 |
| IC | 1178 ± 39 | 1343 ± 48 | 963 ± 42 | 1117 + 49 |

Dans le tableau 8, le taux d'incorporation est mesuré par le nombre de désintégration de la 3H-hydroxyproline radioactive par minute (dpm) par mg de masse sèche. Plus ce nombre est élevé, plus la concentration en 3H-hydroxyproline est élevée.

Les résultats du tableau 8 montrent que la catéchine et le complexe de chlorhydrolysinate de (+)-catéchine stimulent la biosynthèse du tissu conjonctif et particulièrement celle du collagène.

En outre, cet exemple confirme la protection de la trame conjonctive *in vivo* par l'absorption orale des complexes de (+)-catéchine monomérique par voie orale et dès lors la protection de cette trame notamment contre l'intrusion de cellules cancéreuses.

**Exemple comparatif 5: mesure de l'action de la (+)-catéchine monomérique pure sur l'incorporation totale et dans le collagène de la 3H-proline au niveau de la peau**

Dans cet exemple comparatif, un groupe E de 12 rats Wistar pesant de 95 à 115 g auxquels de la 3H-hydroxyproline radioactive, dont l'activité spécifique est de 13,6 Ci/$10^{-3}$ mol, a été administrée à l'ensemble des individus par injection intrapéritonéale de proline-L-(5-3H) à une dose par rapport au poids des individus de 1mCi/kg, ce qui représente une activité en 3H-proline radioactive de 100 μCi/individu.

6 rats de chaque groupe ont reçu un traitement à base de (+)-catéchine monomérique pure par gavage à raison de 100mg/kg pendant 5 jours avant le sacrifice. Les 6 autres rats n'ayant pas reçu de traitement à base de (+)-catéchine monomérique est un sous-groupe de contrôle (ctrl).

Tableau 9 : action de la (+)-catéchine monomérique pure (100 mg/kg) sur le taux (en dpm/mg) d'incorporation totale (IT) et dans le collagène (IC) de 3H-hydroxyproline dans la peau de rat

| Taux | Groupe | |
| | E | |
| | ctrl | traité |
|---|---|---|
| IT | 4864 ± 231 | 5689 ± 410 |
| IC | 963 ± 42 | 1124 ± 51 |

Dans le tableau 9, le taux d'incorporation est mesuré par le nombre de désintégration de la 3H-hydroxyproline radioactive par minute (dpm) par mg de masse sèche. Plus ce nombre est élevé, plus la concentration en 3H-hydroxyproline est élevée.

Les résultats des tableaux 8 et 9 montrent que l'augmentation du taux d'incorporation pour les complexes de chlorhydrolysinate de (+)-catéchine monomérique (+15%) et de l'ascorbolysinate de (+)-catéchine monomérique (+14%) est équivalent à celui mesuré lorsque les rats sont gavés à la (+)-catéchine monomérique pure (+16%).

Cet exemple comparatif confirme la protection de la trame conjonctive *in vivo* par l'absorption orale des complexes de (+)-catéchine monomérique par voie orale et dès lors la protection de cette trame notamment contre l'intrusion de cellules cancéreuses, et ce, à des teneurs en (+)-catéchine monomérique *de facto* plus faible (61 mg de (+)-catéchine pour 100 mg de complexe) que celle rencontrée lors de la prise de (+)-catéchine pure (100mg de (+)-catéchine).

**Exemple** 5 : **mesure de l'action du chlorhydrolysinate de (+)-catéchine monomérique (sel de complexe [C: Lys : HCl] sur l'incidence d'ulcères gastriques et sur le taux d'histamine dans la muqueuse gastrique chez le rat Mastomys**

Dans cet exemple, l'efficacité du chlorhydrolysinate de (+)-catéchine monomérique *in vivo* chez un rat africain, le Mastomys qui est connu pour faire spontanément des lésions gastriques et notamment des carcinomes gastriques est mise en évidence.

Le Mastomys a en effet un système spécial de cellules stockant l'histamine au niveau de la muqueuse gastrique, ces cellules peuvent donner naissance à des tumeurs carcinoïdes.

Dans cet exemple, un échantillon de n=48 Mastomys sont sensibilisés une première fois par une injection intrapéritonéale de 3 $\mu$g d'ovalbumine dissoute dans 0,2ml de solution saline contenant 1mg d'hydroxyde d'aluminium l'ovalbumine ce qui provoque des anticorps anti-immunoglobulines E(lgE) et G(lgG). Après 7 jours une seconde injection de 1mg d'ovalbumine dans 0,01ml de solution saline est effectuée dans la muqueuse gastrique au niveau du corpus des animaux anesthésiés, ce qui provoque une lésion ulcéreuse au niveau même de l'injection.

Les 48 rats sont divisés en trois groupes : un de 24 et deux de 12 individus. Le premier groupe de 24 individus (G1) a reçu un placebo (solution de NaCl concentrée à du 0.15 M).

Le second groupe (G2) de 12 individus a été traité par le chlorhydrolysinate de (+)-catéchine monomérique administré *per* os à raison de deux fois par jour (2 $\times$ 300 mg), deux jours avant l'injection et trois jours après injection. Le troisième groupe (G3) de 12 individus ont reçu, dans les mêmes conditions que celle du groupe G2, le chlorhydrolysinate de (+)-catéchine monomérique à raison de 100 mg mais par voie intrapéritonéale.

Comme le montre les résultats de le tableau 10, l'administration du chlorhydrolysinate de (+)-catéchine par voie orale donne une diminution très significative (-72%) du nombre d'animaux présentant un ulcère gastrique (NU) ainsi qu'une diminution significative (-18%) du taux d'histamine (TH), ce taux étant exprimé en $10^{-6}$ mg/kg de protéine, dans la muqueuse gastrique. Le taux d'histamine est mesuré pour les groupes G1, G2 et G3 sur base d'un échantillon de n=12 individus.

La diminution du taux d'histamine accumulée au niveau de la muqueuse gastrique résulte directement de l'action de la (+)-catéchine monomérique sur la paroi des cellules qui stockent l'histamine. En contribuant au renforcement de la paroi de ces cellules, la (+)-catéchine monomérique permet de diminuer le taux d'histamine assimilées au niveau de la muqueuse gastrique et donc de réduire le nombre d'ulcères.

Il est intéressant de noter par rapport à cette expérience que notamment dans de nombreux types de leucémies, chroniques ou aigües, on retrouve une augmentation des mastocytes avec augmentation concomitante des taux d'histamine ainsi qu'une augmentation des taux d'immunoglobulines (Blood Cells Mol. Dis. 35 (3), 370-383, 2005).

Tableau 10 : action du chlorhydrolysinate de (+)-catéchine sur l'incidence d'ulcères gastriques et sur le taux d'histamine dans la muqueuse gastrique chez le Mastomys

| Groupe | n | NU | TH (n=12) |
|---|---|---|---|
| G1 | 24 | 23 | 46,17 $\pm$ 2,30 |
| **G2** | **12** | **5** | **37,91 $\pm$ 3,73** |
| G3 | 12 | 6 | 34,98 $\pm$ 3,52 |

**Exemple 6** : **mesure de l'action du chlorhydrolysinate de (+)-catéchine monomérique (sel de complexe [C : Lys : HCl] dans le traitement de patients atteints du cancer**

Dans cet exemple, les résultats relatifs au test de l'effet du chlorhydrolysinate de (+)-catéchine monomérique chez deux patients cancéreux traités pendant plus d'un an à raison d'un comprimé de 500 mg de sel de complexe [C : Lys : HCl] par jour sont présentés.

Chez le premier patient, un homme de 67 ans, un lymphome lymphoplasmocytique (de type macroglobulinémie de Waldenstöm) a été détecté lors d'une analyse sanguine post opératoire au jour $J_0$. Le diagnostic se base sur la détection à l'électrophorèse d'un pic monoclonal d'immunoglobulines (IgM). Ce diagnostic a été reconfirmé et le pic dosé lors d'une seconde analyse utilisant la même détection par électrophorèse.

Les immunoglobulines jouent un rôle essentiel dans la défense de l'organisme contre les agressions et sont normalement sécrétées par les lymphocytes B.

Dans le myélome multiple ou par exemple dans le cas de la maladie de Waldenstöm, on observe une sécrétion d'un seul type d'immunoglobuline ou immunoglobuline monoclonale par des plasmocytes présents dans la moelle osseuse et qui prolifèrent d'une manière incontrôlée.

On retrouve ces immunoglobulines à une concentration élevée dans le sang et dans les urines. Elles constituent donc de véritables marqueurs tumoraux. Leur dosage rend compte du nombre de cellules malades, de l'extension de la maladie et par conséquent permet de suivre son évolution sous traitement.

Les contrôles sanguins successifs ont donné les données suivantes pour le dosage d'IgM (en mg pour 100 ml de plasma) :

Tableau 11 : évolution du taux d'IgM (en mg/100 ml) dans le sang au cours du temps

| Période de prélèvement | IgM |
|---|---|
| $J_0$ | 1910 |
| $J_0$ + 1 mois | 2010 |
| $J_0$ + 5 mois | 1966 |
| $J_0$ + 8 mois | 2467 |
| $J_0$ + 12 mois | 2280 |
| $J_0$ + 15 mois | 2748 |
| $J_T$ | 2931 |
| $J_T$ + 4 mois | 2417 |
| $J_T$ + 9 mois | 2410 |
| $J_T$ + 15 mois | 2412 |

Les résultats du tableau 11 montrent qu'il y a, depuis la prise de chlorhydrolysinate de (+)-catéchine monomérique pendant un an et demi, une stabilisation en dessous du maximum détecté au jour $J_T$ à partir duquel le traitement a été administré.

Le taux d'infiltration est resté faible et malgré des valeurs d'IgM supérieure à 2000 mg/100 ml. Le patient n'a en outre pas subi de traitement chimiothérapeutique. On considère donc qu'il est stabilisé.

Chez le second patient, une adénopathie cervicale sous-claviculaire gauche a tout d'abord été dépistée au jour $J_0$. Ensuite, 2 semaines plus tard, un adénocarcinome rénal gauche a été détecté pour ce patient, suivi d'une néphrectomie du rein gauche 3 jours après la détection de l'adénocarcinome au niveau de ce rein.

Par après, au jour $J_0$ + 3 mois, un curage ganglionnaire cervical gauche a été appliqué au patient. Après cette intervention, s'en est suivie une série de 15 séances de radiothérapie.

A partir du jour $J_0$ + 15 mois (soit ou jour $J_T$), le patient s'est vu administré par voie orale un comprimé de 500mg de chlorhydrolysinate de (+)-catéchine par jour.

Au jour $J_T$ + 15 mois, l'apparition d'une adénopathie médiastinale pathologique (d'un volume de 45 $cm^3$) a été constatée et a nécessité un curage ganglionnaire médiastinale suivi d'une exploration pulmonaire au jour $J_T$ + 17 mois. Cet examen a mis en évidence l'involution d'une petite opacité micronodulaire de 5mm dans le poumon gauche et l'absence de lésion secondaire au niveau du parenchyme pulmonaire et de l'étage abdominal.

A $J_T$ + 19 mois, le rapport de consultation a conclu que le carcinome rénal à cellules claires était en rémission complète. Ce diagnostic a été reconfirmé lors des examens de contrôle effectués à $J_T$ + 23 mois. Clairement, il apparaît que la prise de chlorhydrolysinate de (+)-catéchine pendant un an ait stabilisé l'état du patient.

*Synthèse d'une composition de précurseurs d'un complexe ou d'un sel de complexe*

Le procédé de fabrication d'une composition comprenant la (+)-catéchine monomérique et au moins un acide aminé basique en tant que précurseur du complexe comprend les étapes suivantes :

- une amenée d'une première quantité de (+)-catéchine monomérique ;
- une amenée d'une deuxième quantité d'au moins un acide aminé basique, ou d'au moins un dérivé d'acide aminé basique, de sorte à obtenir un ratio d'équivalence molaire entre ladite (+)-catéchine et ledit au moins un acide aminé basique ou ledit au moins un dérivé d'acide aminé basique, compris entre 1 : 1 et 1 : 2,5, de préférence supérieur ou égal à 1 : 1, en particulier supérieur à 1 : 1, de préférence inférieur ou égal à 1 : 2,5, en particulier inférieur à 2,5, plus particulièrement inférieur ou égal à 1 : 2, plus particulièrement inférieur à 2 ; et

- une mise en contact de ladite (+)-catéchine monomérique avec ledit au moins un acide aminé basique, ou avec ledit dérivé d'acide aminé basique, de façon à obtenir ledit mélange de (+)-catéchine monomérique et d'au moins un acide aminé basique ou d'au moins un dérivé d'un acide aminé basique.

De manière préférentielle, le procédé comprend une étape supplémentaire qui consiste en une amenée d'un acide, de préférence d'acide ascorbique.

De manière alternative, le procédé comprend une étape supplémentaire qui consiste en une amenée d'une phase aqueuse et en une solubilisation dudit mélange dans ladite phase aqueuse pour former un complexe de (+)-catéchine et d'au moins un acide aminé basique, ledit complexe étant solubilisé dans ladite phase aqueuse.

Alternativement, le procédé comprend en outre une étape d'addition d'un excipient biocompatible audit mélange.

De préférence, ledit au moins un acide aminé amené est sélectionné dans le groupe constitué de la lysine et de l'arginine, d'origine naturelle ou de synthèse, et d'un mélange de ceux-ci.

Dans les exemples comparatifs 6 à 9 qui suivent, la composition administrée par voie orale a été préparée à base d'un mélange de chlorhydrolysinate (Lys: HCl) sous forme pulvérulente avec de la (+)-catéchine pure monomérique aux rapports d'équivalent molaire de 1 : 1, 1 : 2, 1 : 3 et 1 : 5.

On obtient une composition qui comprend la (+)-catéchine monomérique et au moins un acide aminé basique en tant que précurseur dudit sel de complexe [C : Lys : HCl].

Chacun des mélanges est ensuite solubilisé dans de l'eau. Durant la solubilisation du mélange, il y a formation du complexe de (+)-catéchine et de chlorhydrolysinate en phase aqueuse. La solution ainsi obtenue est une solution de chlorhydrolysinate de (+)-catéchine sous forme de complexe : le complexe obtenu dans cette solution est un complexe de (+)-catéchine monomérique et d'au moins un acide aminé basique (ou son dérivé) dans un ratio d'équivalence molaire identique à celui d'un mélange comme décrit. Ainsi, un mélange de (+)-catéchine monomérique et d'un acide aminé à hauteur d'un ratio d'équivalence molaire 1 : 1 donnera, une fois solubilisé dans la phase aqueuse, une solution de complexe de de (+)-catéchine monomérique et d'au moins un acide aminé basique (ou son dérivé) dans un ratio d'équivalence molaire égal à 1 : 1.

Cette solution est ensuite administrée *per os* (PO) ou par injection intraveineuse (IV) à chaque individu d'un groupe de 5 rats Wistar, à raison d'une dose de 25 mg (par kg de poids corporel) de (+)-catéchine monomérique pure ou une dose de lysinate de (+)-catéchine équivalente à 25 mg de (+)-catéchine.

_Biodisponibilité par voie orale de la composition de précurseurs du complexe ou du sel de complexe_

**Exemple comparatif 6 : mesure de la biodisponibilité par voie orale et par voie injectable du complexe de (+)-catéchine et de lysine, à un ratio d'équivalence molaire de 1 : 1 et 1 : 2, chez le rat**

Tableau 12a: concentration plasmatique (cc) de la (+)-catéchine monomérique totale (en ng/ml) en fonction du temps

| T (min) | | | |
|---|---|---|---|
| | IV | | PO |
| T (min) | [C :Lys :HCl/1 : 2 :2] | [C :Lys :HCl/1 : 1 : 1] | [C :Lys :HCl/1 :2 :2] |
| | cc (ng/ml) | | |
| 10 | 8066 | 6406 | 1839 |
| 30 | 2639 | 2259 | 3780 |
| 60 | 1191 | 963 | 3190 |
| 120 | 338 | 270 | 1990 |
| 240 | 0 | 0 | 475 |

Tableau 12b: concentration plasmatique (cc) de la (+)-catéchine monomérique libre (en ng/ml) en fonction du temps T (min)

| T (min) | IV | PO |
|---|---|---|
| | [C :Lys :HCl/1 :2 :2] | [C :Lys :HCl/1 :2 :2] |
| | cc (ng/ml) | |
| 10 | 3340 | 428 |
| 30 | 991 | 1027 |
| 60 | 291 | 1188 |
| 120 | 0 | 767 |
| 240 | 0 | 179 |

Tableau 12c : comparaison des concentrations plasmatiques totales (ct) en ng min/ml de la (+)-catéchine *monomérique* libre et de la (+)-catéchine monomérique totale après administration intraveineuse et orale de 25mg/kg de C :Lys :HCl/ 1 :2 :2 chez le rat.

| | IV | PO |
|---|---|---|
| Libre | 87970 | 165325 ($\Delta$ct* : +88%) |
| Totale | 277040 | 479585 ($\Delta$ct* : +73%) |
| *$\Delta$ct mesuré entre les données IV et PO. | | |

Après injection intraveineuse du sel de complexe [C :Lys :HCl/ 1: 2: 2], les taux plasmatiques (cc) de (+)-catéchine naturellement très élevés au départ retombent très rapidement à des valeurs très faibles, 340 ng/ml après 2 heures pour la (+)-catéchine totale retrouvée dans le plasma et 0 ng/ml pour la (+)-catéchine libre, alors que les taux sanguins totaux du même produit pris par voie orale dans les mêmes conditions se maintiennent après 2 heures à 1990 ng/ml et la quantité de (+)-catéchine libre à 767 ng/ml, comme indiqué dans les résultats repris aux tableaux 12a et 12b.

Cette injection intraveineuse (rendue possible grâce au chlorhydrolysinate rendant la (+)-catéchine soluble) montre, qu'il y ait une ou deux molécules de lysine associées à la (+)-catéchine monomérique, que cette (+)-catéchine est rapidement éliminée de la circulation sanguine.

Par contre l'ingestion par voie orale de la forme complexée de la (+)-catéchine monomérique, d'une part, augmente les quantités de (+)-catéchine monomérique libre dans le sang, et d'autre part, prolonge le pic de concentration maximale.

Ceci est un résultat surprenant. En effet les taux plasmatiques obtenus après injection directe dans le sang donnent des taux sanguins nettement plus élevés sur la première heure après administration que lorsque le produit est donné par voie orale, ce qui est attendu. Par contre, lorsque le complexe est administré *per os,* il est observé des ct en (+)-catéchine monomérique libre augmentés de 88% en ce qui concerne la (+)-catéchine libre disponible dans le plasma (ct de 165325 ng min /ml pour une administration *per os* contre seulement 87970 ng min /ml pour une administration par IV), il en est de même pour la catéchine totale retrouvée dans le plasma, la ct augmente de la même façon de 73% ce qui est tout à fait remarquable. La voie orale se révèle donc de façon surprenante supérieure à la voie intraveineuse pour la biodisponibilité globale, qu'elle soit libre ou totale, de la (+)-catéchine dans le plasma après administration du sel de complexe [C :Lys :HCl/1 :2 :2].

De plus, là où, après 120 min, il n'y a pratiquement plus de (+)-catéchine dans le sang après injection IV, on observe après prise orale du sel de complexe [C :Lys :HCl/ 1 : 2 :2] au même moment les taux maximum de catéchine dans le sang.

Il est donc démontré que le sel de complexe [C :Lys :HCl/ 1 : 2 : 2] améliore la biodisponibilité de la catéchine de façon surprenante, en effet cette biodisponibilité sanguine de la (+)-catéchine, qu'elle soit libre ou conjuguée, est étonnement meilleure par voie orale que par injection intraveineuse.

Ceci montre que les taux et les données de biodisponibilité par voie intraveineuse ne sont pas extrapolables à la voie orale et que ces résultats se différencient clairement du document US 4 285 964 dans lequel aucun test de biodisponibilité pour une prise *per* os n'a été exemplifié.

**Exemple comparatif 7: mesure de l'effet de l'acide sur la biodisponibilité par voie orale du complexe de (+)-catéchine et de lysine chez le rat**

Afin de vérifier, l'effet ou non de l'addition d'un acide au complexe [C: Lys/ 1 : 2], nous avons mesuré les taux en (+)-catéchine monomérique libre dans le plasma après ingestion par voie orale de CP ou du sel de complexe [C : Lys : HCl/ 1 : 2 : 2]. Les résultats sont présentés dans le tableau ci-dessous :

Tableau 13: paramètres de T (max), c (max) et de ct en (+)-catéchine libre présente dans le sang calculés à partir des courbes de concentrations plasmatiques (cc) de la (+)-catéchine monomérique libre (en ng/ml) pour chaque source de (+)-catéchine en fonction du temps T (minute)

|  | Source de (+)-catéchine monomérique | |
| --- | --- | --- |
|  | CP | [C : Lys : HCl/1 : 2 : 2] |
| T (max) (min) | 60 | 60 |
| c (max) (ng/ml) | 580 | 1200 |
| ct (ng min/ml) | 91935 | 165325 |
| Δct (en %) | 0 | +77 |

On retrouve l'effet positif des 2 molécules de lysine sur la biodisponibilité de la (+)-catéchine monomérique et sur sa concentration maximale, l'addition de 2 molécules de HCl influence l'effet des 2 molécules de lysine : Δct de + 77% en présence d'HCl et de +17% sans HCl (voir tableau 7a).

**Exemple comparatif 8 : mesure chez le rat de l'effet du ratio d'équivalence molaire entre la (+)-catéchine et l'acide aminé basique sur la biodisponibilité par voie orale du complexe de chlorhydrolysinate de (+)-catéchine**

Tableau 14 : paramètres de T (max), c (max) et de ct en (+)-catéchine libre présente dans le sang calculés à partir des courbes de concentrations plasmatiques (cc) de la (+)-catéchine monomérique libre (en ng/ml) pour chaque source de (+)-catéchine en fonction du temps T (minute)

|  | Source de (+)-catéchine monomérique | | | | | |
| --- | --- | --- | --- | --- | --- | --- |
|  | CP | ratio | | | | |
|  |  | 1 : 2 : 2 | 1 : 2,5 : 2,5 | 1 : 3 : 3 | 1 : 5 : 5 | 1 : 2 : 2§ |
| T (max) (min) | 60 | 60 | - | 60 | 60 | 60 |
| c (max) (ng/ml) | 580 | 1200 | - | 280 | 265 | 226 |
| ct (ng min/ml) | 91935 | 165325 | 105500* | 38000 | 31136 | 44095 |
| Δct (en %) | 0 | +80 | +15 | -59 | -66 | -48 |
| * valeur de ct estimée à partir du profil de la courbe d'évolution du ct en fonction du ratio d'équivalence molaire (+)-catéchine monomérique : Lysine (voir figure 5) .Les autres valeurs de ct estimées au départ de la figure 5 sont celles du ratio 1 :1,5 :1,5. qui est de 142000 ng min/ml et du ratio 1 :1 :1 qui est de 127000 ng min/ml.<br>§ prise *per* os séparée du chlorhydrolysinate et de la (+)-catéchine pure. | | | | | | |

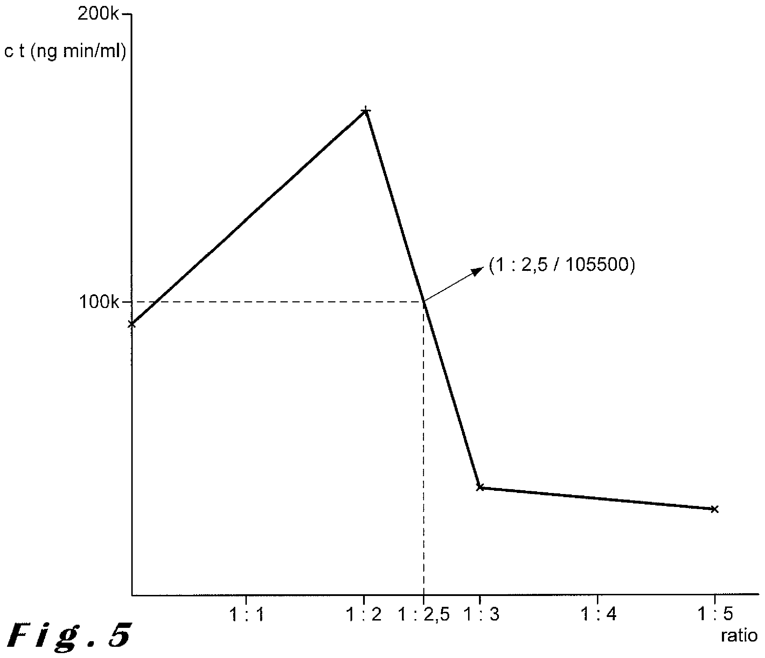

La valeur du ct (ng min/ml) pour le sel de complexe C :Lys :HCL / 1 :1,5 :1,5 reportée sur la courbe figure 5 est de 142k ng min/ml, soit meilleure que pour 1 :1 :1 (127k,valeur reportée) et meilleure que pour 1 :2,5 :2,5 (105k ng min/ml, valeur reportée) mais moins bonne que pour 1 :2 :2 (165k, valeur calculée).

Toutes les valeurs de ct obtenues pour des sels de (+)-catéchine en proportions égales ou inférieures à 1 :2,5 :2,5 sont nettement meilleures que les valeurs obtenues pour ces mêmes sels en proportions supérieures, 1 :3 :3 (38k, valeur calculée) et 1 :5 :5 (26k, valeur calculée).

Ces résultats confirment les résultats de l'exemple comparatif 4 : le sel de complexe [C :Lys :HCL/ 1 : 2 : 2] est la seule association (+)-catéchine - lysine qui améliore clairement la biodisponibilité par voie orale de la (+)-catéchine.

Les données de cet exemple montrent que non seulement le sel de complexe [C :Lys :HCL/ 1: 2: 2] donne un optimum de biodisponibilité, mais aussi que, dès lors que cette proportion est dépassée, l'effet est clairement inversé, avec un Δct de -59% pour le ratio d'équivalence molaire de 1 : 3 : 3 et de -66% pour le ratio d'équivalence molaire de 1 : 5 : 5.

En outre, le fait d'ingérer séparément (dans un intervalle de temps compris entre 5 et 10 min) la (+)-catéchine monomérique et le chlorhydrolysinate donne un effet négatif (avec un Δct de -48%) sur la quantité de (+)-catéchine monomérique libre dans le plasma. Ce qui démontre que la catéchine et la lysine doivent être mélangés avant ingestion (sous forme solubilisée dans de l'eau ou non), ou du moins, ingérées de manière simultanée.

**Exemple comparatif 9: mesure chez le rat de la nature de la composition thérapeutique sur la biodisponibilité par voie orale**

Que la composition administrée *per os* soient à base du complexe [C :AA/1 : 1 - 1 : 2,5] préparé selon la méthode décrite dans l'exemple 1a (1b) ou 2a (2b) (voir exemples 1 et 2), ou bien que la composition administrée *per os* soit un mélange en ratio d'équivalence molaire désiré de poudre de (+)-catéchine et de chlorhydrate de lysine (dénommé encore sous le nom de chlorydrolysinate), les résultats au niveau des biodisponibilités par voie orale sont similaires (voir tableau 15 ci-dessous).

Compte tenu des résultats de l'exemple comparatif 2 dans lequel le complexe [C :Lys/1 : 2] a été préparé selon le protocole 1a (voir exemple 1), et des résultats de l'exemple comparatif 8, où le sel de complexe [C :Lys : HCl/1 : 2 : 2] a été préparé par simple mélange d'une mole de (+)-catéchine pulvérulente et de deux moles de chlorhydrolysinate pulvérulente et puis mis en solution avant administration *per os,* les résultats résumé au tableau 15 montrent que l'amélioration de biodisponibilité par voie orale pour ces complexes restent dans le même ordre de grandeur.

Tableau 15: paramètres de T (max), c (max) et de ct en (+)-catéchine libre présente dans le sang calculés à partir des courbes de concentrations plasmatiques (cc) de la (+)-catéchine monomérique libre (en ng/ml) pour chaque source de (+)-catéchine en fonction du temps T (minute)

|  | Source de (+)-catéchine monomérique | |
|---|---|---|
|  | [C : Lys/1 : 2] | [C : Lys : HCl /1 : 2 : 2] |
| T (max) (min) | 120 | 60 |
| c (max) (ng/ml) | 1547 | 1200 |
| ct (ng min/ml) | 186348 | 165325 |
| Δct (en %) | +94 | +80 |

Le fait de pouvoir obtenir des paramètres optimum de biodisponibilité sur base d'un mélange pulvérulent de (+)-catéchine et d'acide aminé représente un avantage en termes de coût de production dès lors que la préparation de ce mélange est réalisé en une seule phase solide et de manière très aisée, ce qui permet d'envisager des perspectives de production à grande échelle.

**Exemple comparatif 10 : mesure chez le rat de la biodisponibilité par voie orale pour la quercétine et l'épigallocatéchine gallate (EGCG)**

Dans cet exemple, il est démontré que l'effet de la lysine n'est pas significatif, ni sur la biodisponibilité par voie orale de la quercétine, ni sur celle de l'épigllocatéchine gallate (EGCG), comme le montre les résultats du tableau 16 ci-dessous :

Tableau 16 : paramètres de T (max), c (max) et de ct en polyphénol libre présent dans le sang calculés à partir des courbes de concentrations plasmatiques (cc) du polyphénol (en ng/ml) pour chaque source polyphénol en fonction du temps T (minute)

|  | Source de (+)-catéchine monomérique | |
|---|---|---|
|  | QP | [Q :Lys :HCl/1 : 2 : 2] [§] |
| T (max) (min) | 60 | 60 |
| c (max) (ng/ml) | 10430 | 8777 |
| ct (ng min/ml) | 1311645 | 1541945 |

(suite)

| | Source de (+)-catéchine monomérique | |
| --- | --- | --- |
| | QP | [Q :Lys :HCl/1 : 2 : 2] § |
| $\Delta ct^q$ (en %)** | 0 | +18 |

* QP : quercétine pure, c'est-à-dire non complexée avec au moins un acide aminé basique ;

§ [Q :Lys :HCl/1 : 2 :2]: complexe de quercétine avec deux lysines.

** $\Delta ct^q$: mesure de la différence entre la ct obtenue avec le complexe et la ct mesurée pour la QP, ramenée à la valeur de biodisponibilité pour la QP.

Dans cet exemple, la composition administrée par voie orale a été préparée à base d'un mélange de chlorhydrolysinate (Lys : HCl) sous forme pulvérulente avec de la quercétine ou de l'EGCG pure monomérique aux ratios d'équivalence molaire de 1 : 2 pour la quercétine, et de 1 : 1, 1 : 2, 1 : 3 et 1 : 5, en ce qui concerne l'EGCG.

Chacun des mélanges est solubilisé dans de l'eau. Cette solution est ensuite administrée *per os* (PO) à chaque individu d'un groupe de 5 rats Wistar, à raison d'une dose de 25 mg (par kg de poids corporel) de quercétine ou d'EGCG monomérique pure ou une dose de lysinate de quertécine ou d'EGCGC équivalente à 25 mg de polyphénol.

En ce qui concerne l'EGCG, les taux mesurés restent inférieurs aux limites fiables de détection (c'est-à-dire en dessous de 250 ng/ml) que ce soit pour l'EGCG pur ou pour ses formes complexées, quelle que soit la proportion de chlorhydrolysinate ajouté à l'épigallocatéchine gallate (dans un ratio d'équivalence molaire de 1 : 1 : 1, de 1 : 2 : 2, de 1 : 3 : 3, ou encore de 1 : 5 : 5).

En conclusion, au vu des résultats de biodisponibilité optimum obtenu pour un complexe entre la (+)-catéchine et l'acide aminé basique à ratio d'équivalence molaire 1: 2, et compte tenu du fait que les tentatives de complexation d'autres polyphénols acides comme la quertécine ou l'EGCG aient échoués, il est clairement démontré que les complexes [C: AA/1 : 1 - 1 : 2,5] sont des complexes présentant une activité spécifiques, en ce qui concerne son passage à travers le tractus gastro-intestinal. Clairement, il ne s'agit donc pas d'une simple neutralisation acide-base, qui aurait dû dans ce dernier cas, favoriser aussi la biodisponibilité par voie orale de la quercétine et de l'EGCG.

Il est en outre entendu que la présente invention n'est en aucun cas limitée aux formes particulières de réalisation susmentionnées et que bien des modifications peuvent y être apportées sans sortir du cadre des revendications annexées.

**Revendications**

1.   Composition gastro-entérique à administration orale comprenant un composé sous forme de complexe de (+)-catéchine monomérique et d'au moins un acide aminé basique, ladite composition étant **caractérisée en ce que** ledit composé présente un ratio d'équivalence molaire entre ladite (+)-catéchine monomérique par rapport audit au moins un acide aminé basique compris entre 1 : 1,5 et 1 : 2,5.

2.   Composition selon la revendication 1, dans laquelle ledit ratio d'équivalence molaire est égal à 1 : 2.

3.   Composition selon l'une quelconque des revendications précédentes, dans laquelle ledit au moins un acide aminé basique est de la lysine ou de l'arginine d'origine naturelle ou de synthèse, ou un mélange des deux, avantageusement dans laquelle ledit au moins un acide aminé basique est de la lysine.

4.   Composition selon l'une quelconque des revendications précédentes, **caractérisée en ce que** ledit complexe est sous forme de sel dudit complexe, ledit sel comprenant ledit complexe, ledit complexe comprenant au moins un proton issu d'au moins un acide et au moins un anion issu dudit au moins un acide, ledit sel présentant ledit proton en quantité équimolaire par rapport à celle en acide aminé basique.

5.   Composition selon la revendication 4, dans laquelle ledit acide est choisi parmi l'acide ascorbique, l'acide acétique, l'acide citrique et l'acide chlorhydrique.

6.   Composition selon les revendications 4 ou 5, dans laquelle ledit acide est de l'acide ascorbique.

7.   Composition selon les revendications 4 ou 5, dans laquelle ledit sel dudit complexe est le chlorhydrolysinate de (+)-catéchine monomérique.

8. Composition selon l'une quelconque des revendications précédentes, **caractérisée en ce qu'**elle comprend en outre un ou plusieurs excipients biocompatibles.

9. Composition selon l'une quelconque des revendications précédentes, dans laquelle la teneur en complexe de (+)-catéchine avec ledit au moins un acide aminé basique est comprise entre 15 et 95% en poids par rapport au poids total de ladite composition, de préférence entre 60 et 90%, avantageusement de 65 à 85%.

10. Composition selon l'une quelconque des revendications précédentes, sous forme solide, en particulier sous forme de poudre, de comprimé, ou de tablette.

11. Composition solide gastro-entérique à administration orale comprenant de la (+)-catéchine monomérique et au moins un acide aminé basique, et éventuellement au moins un acide, en tant que précurseurs d'un complexe de ladite (+)-catéchine monomérique et dudit au moins un acide aminé basique ou d'un sel dudit complexe, comme préparation combinée pour une utilisation simultanée, ledit complexe se formant post-administration orale, ladite (+)-catéchine monomérique et ledit au moins un acide aminé basique étant présents dans un ratio d'équivalence molaire compris entre 1 : 1,5 et 1 : 2,5, de préférence égal à 1 : 2.

12. Composition solide gastro-entérique à administration orale comprenant de la (+)-catéchine monomérique et au moins un acide aminé basique, et éventuellement au moins un acide, en tant que précurseurs d'un complexe de ladite (+)-catéchine monomérique et dudit au moins un acide aminé basique ou d'un sel dudit complexe, comme préparation combinée pour une utilisation simultanée en solution dans une phase aqueuse, ledit complexe se formant pré-administration orale dès lors que la composition est mise en solution aqueuse, ladite (+)-catéchine monomérique et ledit au moins un acide aminé basique étant présents dans un ratio d'équivalence molaire compris entre 1 : 1,5 et 1 : 2,5, de préférence égal à 1 : 2.

13. Composition selon l'une quelconque des revendications 1 à 9, sous forme liquide.

14. Composition selon la revendication 8, caractérisée, en solution 0,01 molaire à 25°C, par un pH égal ou supérieur à 3, de préférence compris entre 4 et 11, de manière avantageuse entre 4,5 et 9.

15. Composition selon l'une quelconque des revendications 1 à 14, pour une utilisation dans le traitement préventif et/ou curatif du cancer, de préférence du cancer hépatocellulaire, des leucémies, des myélomes, des lymphomes, des cancers du foie, de la prostate, du sein, de l'utérus, des testicules, de la vessie, des reins, des poumons, des bronches, des os, de la bouche, de l'oesophage, de l'estomac, du pancréas, colon-rectal ; des maladies inflammatoires ; de l'arthrose ; des maladies génétiques du tissu conjonctif telles que l'ostéogénèse imparfaite, les maladies de Marfan, d'Ehlers-Danlos, les maladies de Cutis Laxa, la sclérodermie systémique, et autres maladies du tissu conjonctif où le collagène et/ou l'élastine ; de carences en collagène ; des ulcères peptiques ainsi que des allergies gastro-intestinales ; ou du vieillissement cellulaire.

**Patentansprüche**

1. Gastroenterale Zusammensetzung für die orale Verabreichung, umfassend eine Verbindung in Form eines Komplexes von monomerem (+)-Katechin und mindestens einer basischen Aminosäure, wobei die Zusammensetzung **dadurch gekennzeichnet ist, dass** die Verbindung ein Moläquivalentverhältnis zwischen dem monomeren (+)-Katechin in Bezug auf die mindestens eine basische Aminosäure zwischen 1:1,5 und 1:2,5 aufweist.

2. Zusammensetzung nach Anspruch 1, wobei das Moläquivalentverhältnis gleich 1:2 ist.

3. Zusammensetzung nach einem der vorhergehenden Ansprüche, wobei die mindestens eine basische Aminosäure Lysin oder Arginin natürlichen oder synthetischen Ursprungs oder ein Gemisch der beiden ist, wobei vorzugsweise die mindestens eine basische Aminosäure Lysin ist.

4. Zusammensetzung nach einem der vorhergehenden Ansprüche, **dadurch gekennzeichnet, dass** der Komplex in Form eines Salzes des Komplexes vorliegt, wobei das Salz den Komplex umfasst, wobei der Komplex mindestens ein von mindestens einer Säure stammendes Proton und mindestens ein von der mindestens einen Säure stammendes Anion umfasst, wobei das Salz das Proton in äquimolarer Menge in Bezug auf diejenige an basischer Aminosäure aufweist.

**5.** Zusammensetzung nach Anspruch 4, wobei die Säure aus Ascorbinsäure, Essigsäure, Zitronensäure und Salzsäure ausgewählt ist.

**6.** Zusammensetzung nach den Ansprüchen 4 oder 5, wobei die Säure Ascorbinsäure ist.

**7.** Zusammensetzung nach den Ansprüchen 4 oder 5, wobei das Salz des Komplexes monomeres (+)-Katechinhydrochloridlysinat ist.

**8.** Zusammensetzung nach einem der vorhergehenden Ansprüche, **dadurch gekennzeichnet, dass** sie außerdem einen oder mehrere biokompatible Exzipienten enthält.

**9.** Zusammensetzung nach einem der vorhergehenden Ansprüche, wobei der Gehalt an Komplex von (+)-Katechin mit der mindestens einen basischen Aminosäure zwischen 15 und 95 Gew.-%, bezogen auf das Gesamtgewicht der Zusammensetzung, vorzugsweise zwischen 60 und 90 Gew.-%, vorteilhafterweise zwischen 65 und 85 Gew.-%, beträgt.

**10.** Zusammensetzung nach einem der vorhergehenden Ansprüche in fester Form, insbesondere in Form eines Pulvers, einer Tablette oder einer Pastille.

**11.** Feste gastroenterale Zusammensetzung für die orale Verabreichung, umfassend monomeres (+)-Katechin und mindestens eine basische Aminosäure und gegebenenfalls mindestens eine Säure als Vorstufen eines Komplexes aus dem monomeren (+)-Katechin und der mindestens einen basischen Aminosäure oder eines Salzes des Komplexes als kombinierte Zubereitung für die gleichzeitige Verwendung, wobei der Komplex sich nach der oralen Verabreichung bildet, wobei das monomere (+)-Katechin und die mindestens eine basische Aminosäure in einem Moläquivalentverhältnis zwischen 1:1,5 und 1:2,5, vorzugsweise gleich 1:2, vorliegen.

**12.** Feste gastroenterale Zusammensetzung für die orale Verabreichung, umfassend monomeres (+)-Katechin und mindestens eine basische Aminosäure und gegebenenfalls mindestens eine Säure als Vorstufen eines Komplexes aus dem monomeren (+)-Katechin und der mindestens einen basischen Aminosäure oder eines Salzes des Komplexes als kombinierte Zubereitung für die gleichzeitige Verwendung in Lösung in einer wässrigen Phase, wobei der Komplex sich vor der oralen Verabreichung bildet, sobald die Zusammensetzung in die wässrige Lösung eingebracht wird, wobei das monomere (+)-Katechin und die mindestens eine basische Aminosäure in einem Moläquivalentverhältnis zwischen 1:1,5 und 1:2,5, vorzugsweise gleich 1:2, vorliegen.

**13.** Zusammensetzung nach einem der Ansprüche 1 bis 9 in flüssiger Form.

**14.** Zusammensetzung nach Anspruch 8, **gekennzeichnet durch** einen pH-Wert gleich oder größer als 3, vorzugsweise zwischen 4 und 11, vorteilhafterweise zwischen 4,5 und 9 in 0,01 molarer Lösung bei 25°C.

**15.** Zusammensetzung nach einem der Ansprüche 1 bis 14 für die Verwendung bei der vorbeugenden und/oder kurativen Behandlung von Krebs, vorzugsweise Leberzellkrebs, Leukämien, Myelomen, Lymphomen, Krebserkrankungen der Leber, der Prostata, der Brust, des Uterus, der Hoden, der Harnblase, der Nieren, der Lunge, der Bronchien, der Knochen, des Mundes, der Speiseröhre, des Magens, des Pankreas, von Kolorektalkrebs; Entzündungskrankheiten; Arthrose; genetischen Krankheiten des Bindegewebes, wie Osteogenesis imperfecta, Marfan-Krankheit, Ehlers-Danlos-Krankheit, der Krankheiten Cutis laxa, systemische Sklerodermie, und anderer Krankheiten des Bindegewebes, wobei das Kollagen und/oder das Elastin, Kollagenmängeln; Magengeschwüren sowie gastrointestinalen Allergien oder Zellalterung.

**Claims**

**1.** Gastroenteric composition for oral administration, comprising a compound in the form of a complex of monomeric (+)-catechin and at least one basic amino acid, said composition being **characterized in that** said compound has a molar equivalent ratio of said monomeric (+)-catechin relative to said at least one basic amino acid of between 1:1.5 and 1:2.5.

**2.** Composition according to Claim 1, wherein said molar equivalent ratio is equal to 1:2.

3. Composition according to either one of the preceding claims, wherein said at least one basic amino acid is lysine or arginine of natural or synthetic origin, or a mixture of the two, advantageously wherein said at least one basic amino acid is lysine.

4. Composition according to any one of the preceding claims, **characterized in that** said complex is in the form of a salt of said complex, said salt comprising said complex, said complex comprising at least one proton derived from at least one acid and at least one anion derived from said at least one acid, said salt exhibiting said proton in equimolar amount relative to the amount of basic amino acid.

5. Composition according to Claim 4, wherein said acid is chosen from ascorbic acid, acetic acid, citric acid and hydrochloric acid.

6. Composition according to Claim 4 or 5, wherein said acid is ascorbic acid.

7. Composition according to Claim 4 or 5, wherein said salt of said complex is monomeric (+)-catechin hydrochlorolysinate.

8. Composition according to any one of the preceding claims, **characterized in that** it also comprises one or more biocompatible excipients.

9. Composition according to any one of the preceding claims, wherein the content of complex of (+)-catechin with said at least one basic amino acid is between 15% and 95% by weight relative to the total weight of said composition, preferably between 60% and 90%, advantageously from 65% to 85%.

10. Composition according to any one of the preceding claims, in solid form, in particular in the form of a powder, a tablet or a lozenge.

11. Solid gastroenteric composition for oral administration comprising monomeric (+)-catechin and at least one basic amino acid, and optionally at least one acid, as precursors of a complex of said monomeric (+)-catechin and said at least one basic amino acid or of a salt of said complex, as a combined preparation for simultaneous use, said complex forming post-oral-administration, said monomeric (+)-catechin and at least one basic amino acid being present in a molar equivalent ratio of between 1:1.5 and 1:2.5, preferably equal to 1:2.

12. Solid gastroenteric composition for oral administration comprising monomeric (+)-catechin and at least one basic amino acid, and optionally at least one acid, as precursors of a complex of said monomeric (+)-catechin and said at least one basic amino acid or of a salt of said complex, as a combined preparation for simultaneous use in solution in an aqueous phase, said complex forming pre-oral-administration as soon as the composition is made into an aqueous solution, said monomeric (+)-catechin and said at least one basic amino acid being present in a molar equivalent ratio of between 1:1.5 and 1:2.5, preferably equal to 1:2.

13. Composition according to any one of Claims 1 to 9, in liquid form.

14. Composition according to Claim 8, **characterized, in** 0.01 molar solution at 25°C, by a pH greater than or equal to 3, preferably of between 4 and 11, advantageously between 4.5 and 9.

15. Composition according to any one of Claims 1 to 14, for use in the preventive and/or curative treatment of cancer, preferably of hepatocellular cancer, leukaemias, myelomas, lymphomas, liver cancer, prostate cancer, breast cancer, uterine cancer, testicular cancer, bladder cancer, kidney cancer, lung cancer, bronchial cancer, bone cancer, mouth cancer, oesophageal cancer, stomach cancer, pancreatic cancer, colorectal cancer; inflammatory diseases; arthrosis; connective tissue genetic diseases such as osteogenesis imperfecta, Marfan's disease, Ehlers-Danlos disease, Cutis Laxa disease, systemic scleroderma, and other diseases of the connective tissue wherein collagen and/or elastin; collagen deficiencies; peptic ulcers and also gastrointestinal allergies; or cell aging.

EP 2 996 686 B1

(+)- catéchine ·················
Lysine ——————

6.911
6.904
6.896
6.868
6.831
6.825
6.753
6.684
6.667
6.647

4.329
4.321
4.317
4.251
4.233
4.224
4.207
4.199
3.623
3.603
3.582
3.001
2.977
2.951
2.571
2.544
2.537
2.517
2.491
1.849
1.841
1.827
1.819
1.811
1.794
1.789
1.774
1.767
1.704
1.678
1.653
1.488
1.465
1.438
1.413
1.387

A
B
C

B

A

***Fig. 1a***

3.815

1.126

1.934

3.980
0.688

1.000

3.450
3.912
3.931

7.5   7.0   6.5   6.0   5.5   5.0   4.5   4.0   3.5   3.0   2.5   2.0   1.5   1.0   ppm

*Fig. 1b*

*Fig. 2a*

6.896
6.887
6.860
6.821
6.743
6.664
6.636
6.615
6.609

4.918

4.310
4.199
4.183

3.601
3.582
3.562
3.189
3.166
3.144
2.954
2.918
2.899
2.876
2.548
2.519
2.495
2.467
1.813
1.800
1.778
1.752
1.656
1.634
1.611

3.597

0.267

1.546

2.401

4.266
1.275
0.226
0.137
1.000

8.657

7.5    7.0    6.5    6.0    5.5    5.0    4.5    4.0    3.5    3.0    2.5    2.0    1.5    1.0    ppm

Fig. 2b

*Fig.3*

*F i g . 4a*

Fig. 4b

Fig. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4285964 A
- CH 665634

**Littérature non-brevet citée dans la description**

- **MATA-BILBAO.** *Journal of Agricultural and Food Chemistry,* 2007, vol. 55, 8857
- *Cell Communication and Signaling,* 2010, vol. 8, 22
- *Blood Cells Mol. Dis.,* 2005, vol. 35 (3), 370-383